# EUROPEAN PATENT SPECIFICATION

(11) **EP 1 476 141 B1**
(45) Date of publication and mention of the grant of the patent: **23.04.2008**
(21) Application number: 03742830.7
(22) Date of filing: 19.02.2003
(51) Int. Cl.: A61K 9/70, A61K 31/485, A61K 31/4468, A61K 31/55, A61P 25/04, A61P 25/36, C07D 221/26, C07D 221/28

(54) **TAMPER-RESISTANT TRANSDERMAL OPIOID DELIVERY DEVICES**
MISSBRAUCHSSICHERE TRANSDERMALE OPIOID-VERABREICHUNGSVORRICHTUNGEN
DISPOSITIFS INVIOLABLES D'ADMINISTRATION TRANSDERMIQUE D'OPIOIDE

(30) Priority: 19.02.2002 US 357139 P; 19.02.2002 US 357141 P
(43) Date of publication of application: 17.11.2004
(73) Proprietor: Euro-Celtique S.A., 2330 Luxembourg (LU)
(72) Inventor: SHEVCHUK, Ihor, Yonkers, NY 10710 (US); CASSIDY, James, P., Cortlandt Manor, NY 10567 (US); REIDENBERG, Bruce, Rye, NY 10580 (US); SHARP, Dale, E., Peekskill, NY 10566 (US); KUPPER, Robert, J., East Greenwich, RI 02818 (US)
(74) Representative: Maiwald, Walter
(86) International application number: PCT/US2003/004999
(87) International publication number: WO 2003/070191

(56) References cited:
- WO-A-01/58447
- WO-A-99/32120
- FR-A- 2 184 065
- US-A- 4 668 685
- US-A- 4 769 372
- US-A- 5 352 680
- STINCHCOMB, A.L. ET AL.: "Straight-chain naltrexone ester prodrugs: Diffusion and concurrent estearase biotransformation in human skin" JOURNAL OF PHARMACEUTICAL SCIENCES, vol. 91, no. 12, December 2002 (2002-12), pages 2571-2578, XP001147678
- BURCE, G.L. ET AL.: "Quantitative determination of naltrexone and naltrexone prodrugs by electron-capture gas-liquid chromatography" JOURNAL OF CHROMATOGRAPHY, vol. 137, 1977, pages 323-332, XP009012531
- SUNG, K.C. ET AL.: "Controlled release of nalbuphine prodrugs from biodegradable polymeric matrices: influence of prodrug hydrophilicity and polymer composition" INTERNATIONAL JOURNAL OF PHARMACEUTICS, vol. 172, 1988, pages 17-25, XP002273024
- MAA, Y.F. ET AL.: "CONTROLLED RELEASE OF NALTREXONE PAMOATE FROM LINEAR POLY(ORTHO ESTERS)" JOURNAL OF CONTROLLED RELEASE, ELSEVIER SCIENCE PUBLISHERS B.V. AMSTERDAM, NL, vol. 14, no. 1, 1 September 1990 (1990-09-01), pages 21-28, XP000150660 ISSN: 0168-3659

## Description

### FIELD OF THE INVENTION

This invention relates generally to acyl opiod antagonists or a pharmaceutically acceptable salt thereof and to tamper-resistant transdermal-delivery devices comprising an opioid, or a pharmaceutically acceptable salt thereof, and an acyl opioid antagonist, or a pharmaceutically acceptable salt thereof.

### BACKGROUND OF THE INVENTION

Opioids, also known as opioid agonists, are agents that can be delivered in a transdermal-delivery device.

U.S. Patent No. 4,626,539 to Aungst et al. describes a pharmaceutical composition purportedly useful for transdermal-delivery of an opioid to a mammalian circulation system.

U.S. Patent No. 4,806,341 to Chien et al. describes transdermal-absorption dosage units comprising a backing layer, an adhesive polymer layer, and an adjoining layer of a solid polymer matrix containing a morphinan narcotic analgesic or antagonist and skin penetration enhancers.

U.S. Patent No. 5,069,909 to Sharma et al. describes methods for sustained administration of buprenorphine by its transdermal-delivery from a laminated composite patch.

Drug abusers, however, have learned to defeat certain transdermal-delivery devices. Accordingly, there is a need for more effective methods for deterring opioid abuse while keeping transdermally administered opioids available to patients who have a legitimate need for them.

U.S. Patent No. 5,149,538 to Granger et al. describes a transdermal patch comprising an opioid and an antagonist substance that are separated by an impermeable barrier. The antagonist substance is purportedly releasable from the patch upon being ingested or substantially immersed in a solvent.

U.S. Patent No. 5,236,714 to Lee et al. describes a composition comprising an abusable substance and an amount of antagonist for the abusable substance sufficient to substantially negate the pharmacological effect of the abusable substance. The patent does not disclose an acyl opioid antagonist.

There remains, however, a need in the art for improved transdermal-delivery devices that are effective for preventing abuse yet useful for delivering a therapeutic agent, such as an opioid or a pharmaceutically acceptable salt thereof.

### SUMMARY OF THE INVENTION

The present invention is directed to a transdennal-delivery device comprising an analgesically effective amount of an opioid, or a pharmaceutically acceptable salt thereof, and an acyl opioid antagonist, or a pharmaceutically acceptable salt thereof, in an amount sufficient to inhibit the euphoric effect of the opioid, or pharmaceutically acceptable salt thereof.

The invention further relates to methods for treating or preventing pain in a patient comprising contacting the skin of a patient in need thereof with a transdermal-delivery device comprising an analgesically effective amount of an opioid, or a pharmaceutically acceptable salt thereof, and an acyl opioid antagonist, or a pharmaceutically acceptable salt thereof, in an amount sufficient to inhibit the euphoric effect of the opioid, or pharmaceutically acceptable salt thereof, wherein the contacting is for an amount of time sufficient to treat or prevent pain.

The invention further relates to a compound selected from the group consisting of 14-(acetyl)nalmefine, 3-(acetyl)nalbuphine, 6-(acetyl)nalbuphine, 14-(acetyl)nabuphine, and a pharmaceutically acceptable salt thereof;
8-(phenylacetyl)cyclazocine, 3-(phenylacetyl)naloxone, 14-(phenylacetyl)naloxone, 3-(phenylacetyl)naltrexone, 14-(phenylacetyl)naltrexone, 3-(phenylacetyl)levallorphan, 3-(phenylacetyl)nalmefene, 14-(phenylacetyl)nalmefene, 3-(phenylacetyl)nalbuphine, 6-(phenylacetyl)nalbuphine, 14-(phenylacetyl)nalbuphine, 6-(phenylacetyl)nalorphine, 3-(phenylacetyl)nalorphine, and a pharmaceutically acceptable salt thereof;
8-(tartaryl)cyclazocine, 3-(tartaryl)naloxone, 14-(tartaryl)naloxone, 3-(tartaryl)naltrexone, 14-(tartaryl)naltrexone, 3-(tartaryl)levallorphan, 3-(tartaryl)nalmefene, 14-(tartaryl)nalmefene, 3-(tartaryl)nalbuphine, 6-(tartaryl)nalbuphine, 14-(tartaryl)nalbuphine, 6-(tartaryl)nalorphine, 3-(tartaryl)nalorphine, and a pharmaceutically acceptable salt thereof;
8-(propionyl)cyclazocine, 3-(propionyl)naloxone, 14-(propionyl)naloxone, 3-(propionyl)naltrexone, 14-(propionyl)naltrexone, 3-(propionyl)nalmefene, 14-(propionyl)nalmefene, 6-(propionyl)nalbuphine, 14-(propionyl)nalbuphine, 6-(propionyl)nalorphine, 3-(propionyl)nalorphine, and a pharmaceutically acceptable salt thereof;
8-(1-glutamyl)cyclazocine, 3-(1-glutamyl)naloxone, 14-(1-glutamyl)naloxone, 3-(1-glutamyl)naltrexone, 14-(1-glutamyl)naltrexone, 3-(1-glutamyl)levallorphan, 3-(1-glutamyl)nahnefene, 14-(1-glutamyl)nalmefene, 3-(1-glutamyl)nalbuphine, 6-(1-glutamyl)nalbuphine, 14-(1-glutamyl)nalbuphine, 6-(1-glutamyl)nalorphine, 3-(1-glutamyl)nalorphine, 8-(5-glutamyl)cyclazocine, 3-(5-glutamyl)naloxone, 14-(5-glutamyl)naloxone, 3-(5-glutamyl)naltrexone, 14-(5-glutamyl)naltrexone, 3-(5-glutamyl)levallorphan, 3-(5-glutamyl)nalmefene, 14-(5-glutamyl)nalmefene, 3-(5-glutamyl)nalbuphine, 6-(5-glutamyl)nalbuphine, 14-(5-glutamyl)nalbuphine, 6-(5-glutamyl)nalorphine, 3-(5-glutamyl)nalorphine, and a pharmaceutically acceptable salt thereof;
8-(benzoyl)cyclazocine; 3-(benzoyl)naloxone; 14-(benzoyl)naloxone; 14-(benzoyl)naltrexone; 3-(benzoyl)levallorphan; 3-(benzoyl)nalmefene; 14-(benzoyl)nalmefene; 6-(benzoyl)nalbuphine; 14-(benzoyl)nalbuphine; 3-(benzoyl)nalorphine; and 14-(benzoyl)nalorphine, wherein the benzoyl group is optionally substituted with a C₁-C₃ alkyl group, -NO₂, -halogen, -OH, or -O(C₁ - C₃ alkyl), and a pharmaceutically acceptable salt thereof;
8-(succinyl)cyclazocine, 3-(succinyl)naloxone, 14-(succinyl)naloxone, 3-(succinyl)naltrexone, 14-(succinyl)naltrexone, 3-(succinyl)levallorphan, 3-(succinyl)nalmefene, 14-(succinyl)nalmefene, 3-(succinyl)nalbuphine, 6-(succinyl)nalbuphine, 14-(succinyl)nalbuphine, 6-(succinyl)nalorphine, 3-(succinyl)nalorphine, and a pharmaceutically acceptable salt thereof;
8-(4-hydroxybutyryl)cyclazocine, 3-(4-hydroxybutyryl)naloxone, 14-(4-hydroxybutyryl)naloxone, 3-(4-hydroxybutyryl)naltrexone, 14-(4-hydroxybutyryl)naltrexone, 3-(4-hydroxybutyryl)levallorphan, 3-(4-hydroxybutyryl)nalmefene, 14-(4-hydroxybutyryl)nalmefene, 3-(4-hydroxybutyryl)nalbuphine, 6-(4-hydroxybutyryl)nalbuphine, 14-(4-hydroxybutyryl)nalbuphine, 6-(4-hydroxybutyryl)nalorphine, 3-(4-hydroxybutyryl)nalorphine, and a pharmaceutically acceptable salt thereof;
8-(glycolyl)cyclazocine, 3-(glycolyl)naloxone, 14-(glycolyl)naloxone, 3-(glycolyl)naltrexone, 14-(glycolyl)naltrexone, 3-(glycolyl)levallorphan, 3-(glycolyl)nalmefene, 14-(glycolyl)nalmefene, 3-(glycolyl)nalbuphine, 6-(glycolyl)nalbuphine, 14-(glycolyl)nalbuphine, 6-(glycolyl)nalorphine, 3-(glycolyl)nalorphine, and a pharmaceutically acceptable salt thereof;
8-(lactyl)cyclazocine, 3-(lactyl)naloxone, 14-(lactyl)naloxone, 3-(lactyl)naltrexone, 14-(lactyl)naltrexone, 3-(lactyl)levallorphan, 3-(lactyl)nalmefene, 14-(lactyl)nalmefene, 3-(lactyl)nalbuphine, 6-(lactyl)nalbuphine, 14-(lactyl)nalbuphine, 6-(lactyl)nalorphine, 3-(lactyl)nalorphine, and a pharmaceutically acceptable salt thereof;
8-(1-aspartyl)cyclazocine, 3-(1-aspartyl)naloxone, 14-(1-aspartyl)naloxone, 3-(1-aspartyl)naltrexone, 14-(1-aspartyl)naltrexone, 3-(1-aspartyl)levallorphan, 3-(1-aspartyl)nalmefene, 14-(1-aspartyl)nalmefene, 3-(1-aspartyl)nalbuphine, 6-(1-aspartyl)nalbuphine, 14-(1-aspartyl)nalbuphine, 6-(1-aspartyl)nalorphine, 3-(1-aspartyl)nalorphine, 8-(4-aspartyl)cyclazocine, 3-(4-aspartyl)naloxone, 14-(4-aspartyl)naloxone, 3-(4-aspartyl)naltrexone, 14-(4-aspartyl)naltrexone, 3-(4-aspartyl)levallorphan, 3-(4-aspartyl)nalmefene, 14-(4-aspartyl)nalmefene, 3-(4-aspartyl)nalbuphine, 6-(4-aspartyl)nalbuphine, 14-(4-aspartyl)nalbuphine, 6-(4-aspartyl)nalorphine, 3-(4-aspartyl)nalorphine, and a pharmaceutically acceptable salt thereof;
8-(sulfanilyl)cyclazocine, 3-(sulfanilyl)naloxone, 14-(sulfanilyl)naloxone, 3-(sulfanilyl)naltrexone, 14-(sulfanilyl)naltrexone, 3-(sulfanilyl)levallorphan, 3-(sulfanilyl)nalmefene, 14-(sulfanilyl)nalmefene, 3-(sulfanilyl)nalbuphine, 6-(sulfanilyl)nalbuphine, 14-(sulfanilyl)nalbuphine, 6-(sulfanilyl)nalorphine, 3-(sulfanilyl)nalorphine, and a pharmaceutically acceptable salt thereof;
8-(fumaryl)cyclazocine, 3-(fumaryl)naloxone, 14-(fumaryl)naloxone, 3-(fumaryl)naltrexone, 14-(fumaryl)naltrexone, 3-(fumaryl)levallorphan, 3-(fumaryl)nalmefene, 14-(fumaryl)nalmefene, 3-(fumaryl)nalbuphine, 6-(fumaryl)nalbuphine, 14-(fumaryl)nalbuphine, 6-(fumaryl)nalorphine, 3-(fumaryl)nalorphine, and a pharmaceutically acceptable salt thereof;
8-(pamoyl)cyclazocine, 3-(pamoyl)naloxone, 14-(pamoyl)naloxone, 3-(pamoyl)naltrexone, 14-(pamoyl)naltrexone, 3-(pamoyl)levallorphan, 3-(pamoyl)nalmefene, 14-(pamoyl)nalmefene, 3-(pamoyl)nalbuphine, 6-(pamoyl)nalbuphine, 14-(pamoyl)nalbuphine, 6-(pamoyl)nalorphine, 3-(pamoyl)nalorphine, and a pharmaceutically acceptable salt thereof;
8-(1-malyl)cyclazocine, 3-(1-malyl)naloxone, 14-(1-malyl)naloxone, 3-(1-malyl)naltrexone, 14-(1-malyl)naltrexone, 3-(1-malyl)levallorphan, 3-(1-malyl)nalmefene, 14-(1-malyl)nalmefene, 3-(1-malyl)nalbuphine, 6-(1-malyl)nalbuphine, 14-(1-malyl)nalbuphine, 6-(1-malyl)nalorphine, 3-(1-malyl)nalorphine, 8-(4-malyl)cyclazocine, 3-(4-malyl)naloxone, 14-(4-malyl)naloxone, 3-(4-malyl)naltrexone, 14-(4-malyl)naltrexone, 3-(4-malyl)levallorphan, 3-(4-malyl)nalmefene, 14-(4-malyl)nahnefene, 3-(4-malyl)nalbuphine, 6-(4-malyl)nalbuphine, 14-(4-malyl)nalbuphine, 6-(4-malyl)nalorphine, 3-(4-malyl)nalorphine, and a pharmaceutically acceptable salt thereof;
8-(maleyl)cyclazocine, 3-(maleyl)naloxone, 14-(maleyl)naloxone, 3-(maleyl)naltrexone, 14-(maleyl)naltrexone, 3-(maleyl)levallorphan, 3-(maleyl)nalmefene, 14-(maleyl)nalmefene, 3-(maleyl)nalbuphine, 6-(maleyl)nalbuphine, 14-(maleyl)nalbuphine, 6-(maleyl)nalorphine, 3-(maleyl)nalorphine, and a pharmaceutically acceptable salt thereof;
of 8-(1-(2-hydroxy)maleyl)cyclazocine, 3-(1-(2-hydroxy)maleyl)naloxone, 14-(1-(2-hydroxy)maleyl)naloxone, 3-(1-(2-hydroxy)maleyl)naltrexone, 14-(1-(2-hydroxy)maleyl)naltrexone, 3-(1-(2-hydroxy)maleyl)levallorphan, 3-(1-(2-hydroxy)maleyl)nalmefene, 14-(1-(2-hydroxy)maleyl)nalmefene, 3-(1-(2-hydroxy)maleyl)nalbuphine, 6-(1-(2-hydroxy)maleyl)nalbuphine, 14-(1-(2-hydroxy)maleyl)nalbuphine, 6-(1-(2-hydroxy)maleyl)nalorphine, 3-(1-(2-hydroxy)maleyl)nalorphine, 8-(4-(2-hydroxy)maleyl)cyclazocine, 3-(4-(2-hydroxy)maleyl)naloxone, 14-(4-(2-hydroxy)maleyl)naloxone, 3-(4-(2-hydroxy)maleyl)naltrexone, 14-(4-(2-hydroxy)maleyl)naltrexone, 3-(4-(2-hydroxy)maleyl)levallorphan, 3-(4-(2-hydroxy)maleyl)nalmefene, 14-(4-(2-hydroxy)maleyl)nalmefene, 3-(4-(2-hydroxy)maleyl)nalbuphine, 6-(4-(2-hydroxy)maleyl)nalbuphine, 14-(4-(2-hydroxy)maleyl)nalbuphine, 6-(4-(2-hydroxy)maleyl)nalorphine, 3-(4-(2-hydroxy)maleyl)nalorphine, and a pharmaceutically acceptable salt thereof;
8-(p-toluenesulfonyl)cyclazocine, 3-(p-toluenesulfonyl)naloxone, 14-(p-toluenesulfonyl)naloxone, 3-(p-toluenesulfonyl)naltrexone, 14-(p-toluenesulfonyl)naltrexone, 3-(p-toluenesulfonyl)levallorphan, 3-(p-toluenesulfonyl)nalmefene, 14-(p-toluenesulfonyl)nalmefene, 3-(p-toluenesulfonyl)nalbuphine, 6-(p-toluenesulfonyl)nalbuphine, 14-(p-toluenesulfonyl)nalbuphine, 6-(p-toluenesulfonyl)nalorphine, 3-(p-toluenesulfonyl)nalorphine, and a pharmaceutically acceptable salt thereof;
8-(stearyl)cyclazocine, 3-(stearyl)naloxone, 14-(stearyl)naloxone, 3-(stearyl)naltrexone, 14-(stearyl)naltrexone, 3-(stearyl)levallorphan, 3-(stearyl)nalmefene, 14-(stearyl)nalmefene, 3-(stearyl)nalbuphine, 6-(stearyl)nalbuphine, 14-(stearyl)nalbuphine, 6-(stearyl)nalorphine, 3-(stearyl)nalorphine, and a pharmaceutically acceptable salt thereof.

The invention further relates to a compound selected from the group consisting of an ester of an α-amino acid formed with 8-hydroxyl group of cyclazocine, the 3- or 14-hydroxyl group of naloxone, the 3- or 14-hydroxyl group of naltrexone, the 3-hydroxyl group of levallorphan, the 3- or 14-hydroxyl group of nalmefene, the 3-, 6-, or 14-hydroxyl group of nalbuphine, the 3- or 6-hydroxyl group of nalorphine, and a pharmaceutically acceptable salt thereof.

The invention further relates to a compound selected from the group consisting of a monoester of a C₅ - C₆ dicarboxylic acid formed with the 8-hydroxyl group of cyclazocine, the 3- or 14-hydroxyl group of naloxone, the 3- or 14-hydroxyl group of naltrexone, the 3-hydroxyl group of levallorphan, the 3- or 14-hydroxyl group of nalmefene, the 3-, 6-, or 14-hydroxyl group of nalbuphine, the 3- or 6-hydroxyl group of nalorphine, and a pharmaceutically acceptable salt thereof.

The present invention may be understood more fully by reference to the following figures, detailed description and examples, which are intended to exemplify non-limiting embodiments of the invention.

### BRIEF DESCRIPTION OF THE DRAWINGS

FIG 1. is a schematic cross-section of a reservoir-type transdermal-delivery device.
FIG 2. is a schematic cross-section of a polymer-matrix transdermal-delivery device.
FIG 3. is a schematic cross-section of a drug-in-adhesive transdermal-delivery device.

### DETAILED DESCRIPTION OF THE INVENTION

The present invention relates to a transdermal-delivery device useful for the transdermal administration of an opioid, or a pharmaceutically acceptable salt thereof, to the systemic circulatory system of a patient and to methods for treating or preventing pain in a patient comprising contacting the skin of a patient in need thereof with the transdermal-delivery device of the invention for an amount of time sufficient to treat or prevent pain. The transdermal-delivery device of the present invention comprises an opioid, or a pharmaceutically acceptable salt thereof, and an acyl opioid antagonist, or a pharmaceutically acceptable salt thereof, in an amount sufficient to inhibit the euphoric effect of the opioid, or a pharmaceutically acceptable salt thereof. In one embodiment, the acyl opioid antagonist, or a pharmaceutically acceptable salt thereof, inhibits the euphoric effect of the opioid, or a pharmaceutically acceptable salt thereof when the opioid, or a pharmaceutically acceptable salt thereof, and the acyl opioid antagonist, or a pharmaceutically acceptable salt thereof, are administered buccally, nasally, parenterally, rectally, and/or vaginally to a patient. In one embodiment, the patient is a human. When contacted with a patient's skin the transdermal-delivery device allows for the transdermal administration of the opioid, or a pharmaceutically acceptable salt thereof, but either (a) allows for the transdermal administration of only an amount of the acyl opioid antagonist, or a pharmaceutically acceptable salt thereof, that is ineffective for inhibiting the analgesic effect of the opioid, or a pharmaceutically acceptable salt thereof, or (b) does not allow the transdermal administration of the acyl opioid antagonist, or a pharmaceutically acceptable salt thereof. But if the transdermal-delivery device of the invention is used to deliver the opioid, or a pharmaceutically acceptable salt thereof, via a route other than transdermal, such as buccal, nasal, oral, parenteral, rectal and/or vaginal, then the acyl opioid antagonist, or a pharmaceutically acceptable salt thereof, blunts or inhibits the euphoric effect of the opioid or pharmaceutically acceptable salt thereof. In one embodiment, the transdermal-delivery device will inhibit the euphoric effect of the opioid, or a pharmaceutically acceptable salt thereof, if used other than transdermally whether before or after the device is used appropriately for treating or preventing pain.

The transdermal-delivery device of the invention is tamper-resistant in that if an abuser attempts to separate the acyl opioid antagonist, or a pharmaceutically acceptable salt thereof, from the opioid, or a pharmaceutically acceptable salt thereof, and self-administer the opioid, or a pharmaceutically acceptable salt thereof, via another route, such as, but not limited to, orally, parenterally, nasally, or buccally, i.e., a route of administration that can result in a quick euphoric rush, also known as the "burst," that abusers prefer, the abuser would self-administer the acyl opioid antagonist, or pharmaceutically acceptable salt thereof, along with the opioid, or pharmaceutically acceptable salt thereof.

For example, if an abuser tries to extract the opioid, or a pharmaceutically acceptable salt thereof, from the transdermal-delivery device by placing it in a solvent, then the acyl opioid antagonist, or a pharmaceutically acceptable salt thereof, would also be extracted, providing a mixture of the opioid, or a pharmaceutically acceptable salt thereof, and the acyl opioid antagonist, or a pharmaceutically acceptable salt thereof. If a mixture of the opioid, or a pharmaceutically acceptable salt thereof, and acyl opioid antagonist, or a pharmaceutically acceptable salt thereof, is administered via a route other than the intended transdermal route, then the acyl opioid antagonist, or a pharmaceutically acceptable salt thereof, would hydrolyze *in vivo* to provide an opioid antagonist, or a pharmaceutically acceptable salt thereof, which exerts its antagonistic effect to inhibit the euphoric effect of the opioid, or a pharmaceutically acceptable salt thereof. In some cases, the acyl opioid antagonist, or a pharmaceutically acceptable salt thereof, is hydrolyzed, i.e., deacylated, during extraction to provide a mixture of the opioid, or a pharmaceutically acceptable salt thereof, and opioid antagonist, or a pharmaceutically acceptable salt thereof.

If the opioid, or a pharmaceutically acceptable salt thereof, and the acyl opioid antagonist, or a pharmaceutically acceptable salt thereof, are administered orally, the acyl opioid antagonist, or a pharmaceutically acceptable salt thereof, is hydrolyzed in the gastrointestinal tract to provide the opioid antagonist, or a pharmaceutically acceptable salt thereof, which exerts its antagonistic effect. If the acyl opioid antagonist, or a pharmaceutically acceptable salt thereof, is not hydrolyzed in the gastrointestinal tract, the opioid, or a pharmaceutically acceptable salt thereof, and acyl opioid antagonist, or a pharmaceutically acceptable salt thereof, are absorbed in the gastrointestinal tract and delivered to the blood stream. There, the acyl opioid antagonist, or a pharmaceutically acceptable salt thereof, becomes hydrolyzed by one or more esterases present in the blood.

Acyl opioid antagonists, or pharmaceutically acceptable salts thereof, that are not hydrolyzed in the gastrointestinal tract but, rather in the blood stream, can be advantageous. Many acyl opioid antagonists, or pharmaceutically acceptable salts thereof, are subject to less first-pass metabolism in the liver that would otherwise occur with the opioid antagonist, or a pharmaceutically acceptable salt thereof *(See, e.g.,* M.A. Hussain et al., Improvement of the Oral Bioavailability of Naltrexone in Dogs: A Prodrug Approach, J. Pharmaceutical Sci., 76(5):356-358 (1987); M.A. Hussain and E. Shefter, Naltrexone-3-Salicylate (a Prodrug of Naltrexone): Synthesis and Pharmacokinetics in Dogs, Pharm. Res., 5(2):113-115 (1988); and U.S. Patent No. 4,668,685. Limiting first pass metabolism in the liver can result in higher bioavailability of the opioid antagonist, or a pharmaceutically acceptable salt thereof, in the blood stream. *Id.*

If the opioid, or a pharmaceutically acceptable salt thereof, and the acyl opioid antagonist, or a pharmaceutically acceptable salt thereof, are administered parenterally, the acyl opioid antagonist, or a pharmaceutically acceptable salt thereof, becomes hydrolyzed in the blood stream via one or more endogenous esterases. Similarly, if the opioid, or a pharmaceutically acceptable salt thereof, and the acyl opioid antagonist, or a pharmaceutically acceptable salt thereof, are administered nasally and absorbed into the blood stream through the nasal mucosa, then the acyl opioid antagonist, or a pharmaceutically acceptable salt thereof, becomes hydrolyzed by one or more esterases present in the blood. If the opioid, or a pharmaceutically acceptable salt thereof, and the acyl opioid antagonist, or a pharmaceutically acceptable salt thereof, are administered buccally, the acyl opioid antagonist, or a pharmaceutically acceptable salt thereof, becomes hydrolyzed by one or more esterases in the saliva. In this case, the opioid antagonist, or a pharmaceutically acceptable salt thereof, becomes absorbed into the blood stream through the buccal mucosa. The acyl opioid antagonist, or a pharmaceutically acceptable salt thereof, can also be absorbed through the buccal mucosa and enter the blood stream, where it is then hydrolyzed via one or more endogenous esterases. Thus, the transdermal-delivery devices of the invention are tamper-resistant and, accordingly, less likely than conventional opioid comprising transdermal-delivery devices to be abused.

The invention further relates to methods for treating or preventing pain in a patient by transdermally administering to the patient in need thereof an analgesically effective amount of an opioid, or a pharmaceutically acceptable salt thereof, with the transdermal-delivery device of the invention.

### DEFINITIONS

The phrase "transdermal-delivery device," as used herein means any device that when contacted with a patient's skin, can transdermally deliver an analgesically effective amount of an opioid, or a pharmaceutically acceptable salt thereof, through the skin to the systemic circulation.

The phrase "pharmaceutically acceptable salt," as used herein, is a salt formed from an acid and the basic nitrogen group of an opioid or an acyl-opiod antagonist. Preferred salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate,*p*-toluenesulfonate, and pamoate *(i.e.,* 1,1'-methylene-bis-(2-hydroxy-3-naphthoate)) salts. The term "pharmaceutically acceptable salt" also refers to a salt prepared from an opioid or an acyl-opiod antagonist having an acidic functional group, such as a carboxylic acid or sulfonic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl,N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N, N,-di-lower alkyl-N-(hydroxy lower alkyl)-amines, such as N, N,-dimethyl-N-(2-hydroxyethyl)amine, or tri-(2-hydroxyethyl)amine; N-methyl-D-glucamine; and amino acids such as arginine, lysine, and the like.

The term "alkyl," as used herein refers to a straight chain or branched, saturated or unsaturated hydrocarbon having from 1 to 8 carbon atoms. Representative alkyls include - methyl, -ethyl, -n-propyl, -n-butyl, -n-pentyl, -n-hexyl, -n-heptyl, -n-octyl, -n-nonly and -n-decyl; while branched alkyls include -isopropyl, -*sec*-butyl, -isobutyl, -*tert*-butyl, -isopentyl, 2-methylbutyl, unsaturated alkyls include -vinyl, -allyl, -1-butenyl, -2-butenyl, - isobutylenyl, -1-pentenyl, -2-pentenyl, -3-methyl-1-butenyl, -2-methyl-2-butenyl, - 2,3-dimethyl-2-butenyl, 1-hexyl, 2-hexyl, 3-hexyl,-acetylenyl, -propynyl, -1-butynyl, - 2-butynyl, =1-pentynyl, -2-pentynyl, -3-methyl-1 butynyl. Alkyl also includes cyclic structures having from 3 to 10 carbon atoms in the ring. Representative cyclic alkyls include -cyclopropyl, -cyclobutyl, -cyclopentyl, -cyclohexyl, -cycloheptyl, -cyclooctyl, - cyclononyl, and -cyclodecyl.

The phrase "fatty acid," as used herein, means any saturated carboxylic acid having from 8 to 16 carbon atoms or any unsaturated carboxylic acid having from 8 to 18 carbon atoms.

The term "acyl opioid antagonist," as used herein means an opioid antagonist having one or more hydroxyl groups, wherein a proton of one of the hydroxyl groups of the opioid antagonist is replaced with an acyl group ("R-C(O)").

The term "1-glutamyl," as used herein means the group:

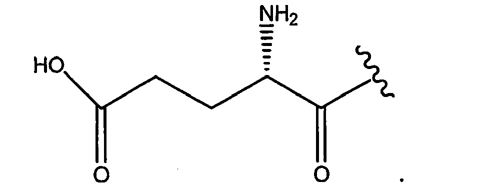

The term "5-glutamyl," as used herein means the group:

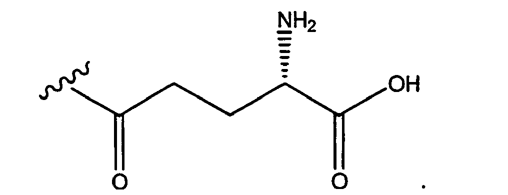

The term "1-aspartyl," as used herein means the group:

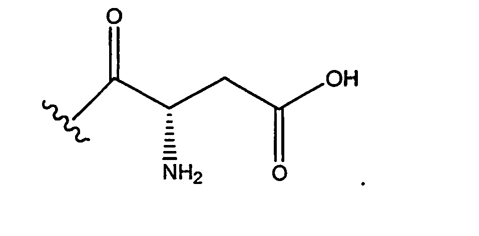

The term "4-aspartyl," as used herein means the group:

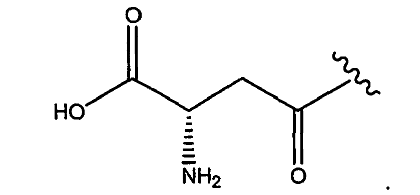

The term "1-malyl," as used herein means the group:

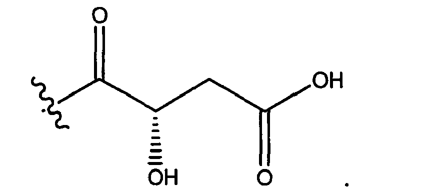

The term "4-malyl," as used herein means the group:

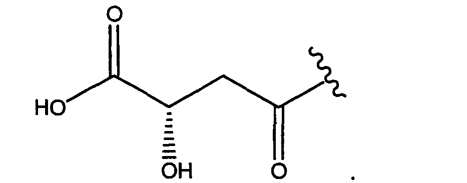

The term "1-(2-hydroxy)maleyl," as used herein means the group:

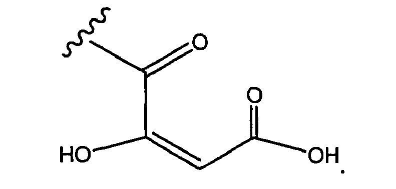

The term "4-(2-hydroxy)maleyl," as used herein means the group:

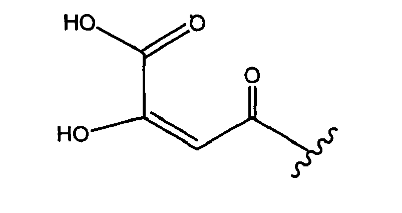

The term "malonyl," as used herein means the group:

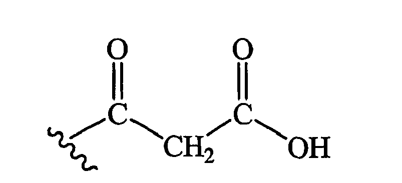

The term "glucuronyl," as used herein means the group:

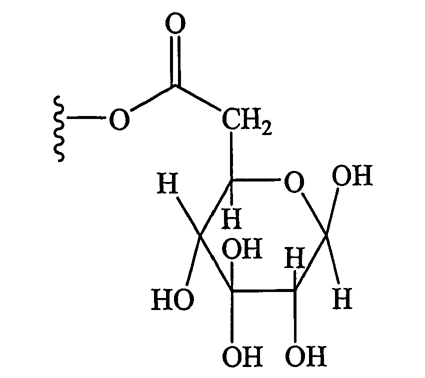

A "patient" is an animal, including, but not limited to, a cow, monkey, horse, sheep, pig, chicken, turkey, quail, cat, dog, mouse, rat, rabbit, chimpanzee, baboon, and guinea pig. In one embodiment, the animal is a mammal. In another embodiment, the animal is a human.

The phrase "treatment of pain" or "treating pain" includes amelioration of pain or the cessation of pain in a patient.

The phrase "prevention of pain" or "preventing pain" includes the avoidance of the onset of pain in a patient.

### THE TRANSDERMAL-DELIVERY DEVICE

Any device known to those skilled in the art for transdermally delivering a therapeutic agent to a patient can be used for the transdermal-delivery device of the invention. For example, the transdermal-delivery device can be a reservoir-type transdermal-delivery device, a polymer-matrix type transdermal-delivery device, or a drug-in-adhesive type transdermal-delivery device *(See, e.g.,* H.S. Tan and W R. Pfister, Pressure Sensitive adhesives for Transdermal Drug Delivery Systems, PSTT, 2(2):60-69 (Feb. 1999).

The transdermal-delivery device is designed so that when contacted with the patient's skin, an analgesically effective amount of the opioid, or a pharmaceutically acceptable salt thereof, is transdermally administered to the patient. But the acyl opioid antagonist, or a pharmaceutically acceptable salt thereof, either remains in the transdermal-delivery device and is not administered to the patient or is administered to the patient in an amount insufficient to inhibit the analgesic effect of the opioid, or a pharmaceutically acceptable salt thereof.

A reservoir-type transdermal-delivery device typically comprises a reservoir, usually a liquid, located between an impermeable backing film and a rate-controlling membrane that is covered with a pressure-sensitive adhesive skin-contacting layer. The reservoir, which can be a solution or a dispersion, contains the opioid, or a pharmaceutically acceptable salt thereof, and the acyl opioid antagonist, or a pharmaceutically acceptable salt thereof. The transdermal-delivery device is supported by the impermeable backing film and the adhesive surface is protected by a release liner. To administer the opioid, or a pharmaceutically acceptable salt thereof, the release liner is removed to expose the pressure-sensitive adhesive and the pressure-sensitive adhesive is contacted with the skin. The opioid, or a pharmaceutically acceptable salt thereof, is permeable through the rate-controlling membrane, and penetrates through it and the adhesive, contacts the skin, and then penetrates the skin. The delivery rate of the opioid, or a pharmaceutically acceptable salt thereof, is usually determined by the rate that the opioid, or a pharmaceutically acceptable salt thereof, penetrates the rate-controlling membrane. In one embodiment, the pressure-sensitive adhesive does not adversely affect the delivery rate of and does not chemically react with the opioid, or a pharmaceutically acceptable salt thereof. The delivery rate is such that an analgesically effective amount of the opioid, or a pharmaceutically acceptable salt thereof, is delivered to the patient. In contrast to the opioid, or a pharmaceutically acceptable salt thereof, however, the acyl opioid antagonist, or a pharmaceutically acceptable salt thereof, which may be present anywhere in the reservoir typically does not penetrate the rate-controlling membrane or, if it does, does so in an amount insufficient to inhibit the analgesic effect of the opioid, or a pharmaceutically acceptable salt thereof.

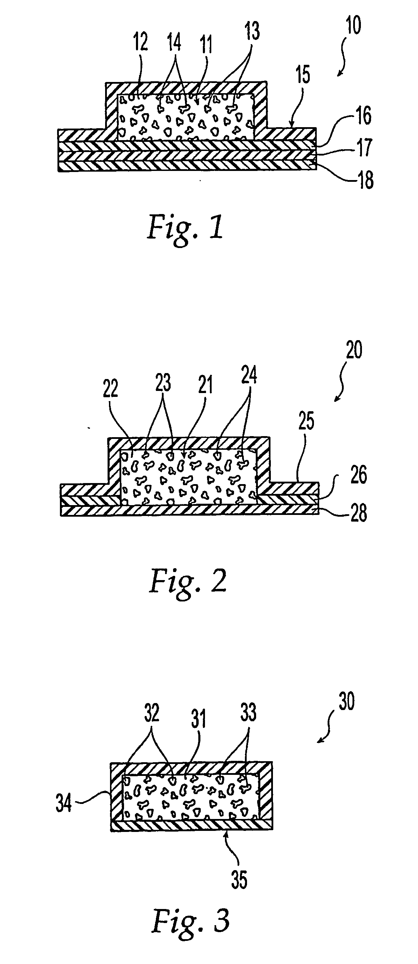

FIG. 1 depicts one embodiment of a reservoir-type transdermal-delivery device. The transdermal-delivery device 10 comprises a reservoir 11, typically in the form of a solution or a dispersion 12, having dispersed therein an opioid, or a pharmaceutically acceptable salt thereof, 13 and an acyl opioid antagonist, or a pharmaceutically acceptable salt thereof, 14. The reservoir 11 is disposed between an impermeable backing film 15, a rate-controlling membrane 16, and a pressure-sensitive adhesive 17. A release liner 18 is applied to the pressure-sensitive adhesive layer 17, and is removed prior to use. In one embodiment, the opioid, or a pharmaceutically acceptable salt thereof, and the acyl opioid antagonist, or a pharmaceutically acceptable salt thereof, are dispersed throughout the reservoir, although uniform dispersion is not necessary.

A variation of the reservoir-type transdermal-delivery system is the polymer-matrix design. In the polymer-matrix design, the opioid, or a pharmaceutically acceptable salt thereof, and the acyl opioid antagonist, or a pharmaceutically acceptable salt thereof, are dispersed in a polymer matrix that controls the delivery rate of the opioid, or a pharmaceutically acceptable salt thereof. Similar to the liquid-reservoir design, the polymer-matrix reservoir is supported on a impermeable backing layer. Rather than having a continuous adhesive layer, however, the polymer-matrix design usually includes a peripheral ring of adhesive located around the edge of the patch. A release liner protects the adhesive surface and the surface of the polymer matrix. To administer the opioid, or pharmaceutically acceptable salt thereof, the release liner is removed to expose the polymer matrix and the ring of pressure-sensitive adhesive, and the device is contacted with the skin. The ring of adhesive holds the device against the skin so that the polymer matrix directly contacts the skin. When the polymer matrix is contacted with the skin, the opioid, or a pharmaceutically acceptable salt thereof, diffuses out of the polymer matrix, contacts the patient's skin, and penetrates the skin. The delivery rate of the opioid agonist, or a pharmaceutically acceptable salt thereof, is usually determined by the rate of diffusion of the opioid, or a pharmaceutically acceptable salt thereof, out of the polymer matrix. The delivery rate is such that an analgesically effective amount of the opioid, or a pharmaceutically acceptable salt thereof, is delivered to the patient. The acyl opioid antagonist, or a pharmaceutically acceptable salt thereof, which may be present anywhere in the polymer matrix, on the other hand, either does not diffuse out of the polymer matrix or, if it does, does so in an amount insufficient to inhibit the analgesic effect of the opioid, or a pharmaceutically acceptable salt thereof.

FIG. 2 depicts a typical polymer-matrix transdermal-delivery device embodiment of the invention. The transdermal-delivery device 20 comprises a reservoir 21 in the form of a polymer matrix 22, having dispersed therein an opioid, or a pharmaceutically acceptable salt thereof, 23 and an acyl opioid antagonist, or a pharmaceutically acceptable salt thereof, 24. In one embodiment, the opioid, or a pharmaceutically acceptable salt thereof, and the acyl opioid antagonist, or a pharmaceutically acceptable salt thereof, are dispersed throughout the polymer matrix, although uniform dispersion is not necessary. The polymer matrix 21 is supported on an impermeable backing layer 25 and has a peripheral ring of adhesive 26 located around the edge of the patch. A release liner 28 is applied to the peripheral ring of adhesive 26 and polymer matrix 22 and is removed prior to use.

The drug-in-adhesive type transdermal-delivery device comprises the opioid agonist, or a pharmaceutically acceptable salt thereof, and the acyl opioid antagonist, or a pharmaceutically acceptable salt thereof, dispersed directly in a pressure-sensitive adhesive matrix. The adhesive matrix is typically supported on the topside with an impermeable backing film and on the side that faces the skin with an impermeable release liner. To administer the opioid, or a pharmaceutically acceptable salt thereof, the release liner is removed to expose the adhesive matrix, and the device is contacted with the skin. The adhesive matrix functions to adhere the device to the skin and, typically, to control the delivery rate of the opioid, or a pharmaceutically acceptable salt thereof. Similar to the polymer-matrix design, the drug-in-adhesive design allows the opioid, or a pharmaceutically acceptable salt thereof, to diffuse out of the adhesive matrix, contact the patient's skin, and penetrate the skin. The delivery rate of the opioid, or a pharmaceutically acceptable salt thereof, is usually determined by the rate of diffusion of the opioid, or a pharmaceutically acceptable salt thereof, out of the adhesive matrix. The delivery rate is such that an analgesically effective amount of the opioid, or a pharmaceutically acceptable salt thereof, is delivered to the patient. The acyl opioid antagonist, or a pharmaceutically acceptable salt thereof, on the other hand, which may be present anywhere in the adhesive matrix, does not diffuse out of the adhesive matrix or does so in an amount insufficient to inhibit the analgesic effect of the opioid, or a pharmaceutically acceptable salt thereof.

FIG. 3 depicts a typical drug-in-adhesive transdermal-delivery device embodiment of the invention. The transdermal-delivery device 30 comprises an adhesive matrix 31 having dispersed there through an opioid, or a pharmaceutically acceptable salt thereof, 32 and an acyl opioid antagonist, or a pharmaceutically acceptable salt thereof, 33. In one embodiment, the opioid, or a pharmaceutically acceptable salt thereof, and the acyl opioid antagonist, or a pharmaceutically acceptable salt thereof, are dispersed throughout the adhesive matrix, although uniform dispersion is not necessary. The adhesive matrix 31 is supported on an impermeable backing layer 34 and has an impermeable release liner 35 on the side that faces the skin which is removed prior to use.

The reservoir type, polymer matrix type, and the drug-in-adhesive type transdermal-delivery systems are well-known to those skilled in the art *(See, e.g.,* H. Tan and W. Pfister, "Pressure Sensitive Adhesives for Transdermal Drug Delivery Systems," PSTT, 2 (February 1999).

Any rate-controlling membrane known to those skilled in the art can be used in the transdermal-delivery device of the invention. In one embodiment, the membrane does not allow any, or any detectable amount, of the acyl opioid antagonist, or a pharmaceutically acceptable salt thereof, to penetrate therethrough, particularly in those instances in which the acyl opioid antagonist, or a pharmaceutically acceptable salt thereof, can penetrate a patient's skin. Suitable materials for the rate-controlling membranes include, but are not limited to, polyethylene; polypropylene; ethylene/propylene copolymers; ethylene/ethylacrylate copolymers; ethylene/vinyl acetate copolymers; polyacrylates; polymethacrylates; silicone elastomers; medical-grade polydimethylsiloxanes; neoprene rubber; polyisobutylene; chlorinated polyethylene; polyvinyl chloride; vinyl chloride-vinyl acetate copolymer; polymethacrylate polymer (hydrogel); polyvinylidene chloride; poly(ethylene terephthalate); butyl rubber; epichlorohydrin rubbers; ethylene-vinyl alcohol copolymer; ethylene-vinyloxyethanol copolymer; silicone copolymers, for example polysiloxane-polycarbonate copolymers, polysiloxane-polyethyleneoxidecopolymers, polysiloxane-polymethacrylate copolymers, polysiloxane-alkylene copolymers (e.g., polysiloxane-ethylene copolymers), polysiloxane-alkylenesilane copolymers (e.g., polysiloxaneethylenesilane copolymers), and the like; cellulose polymers, for example, methyl or ethyl cellulose, hydroxypropyl methyl cellulose, and cellulose esters; polycarbonates; polytetrafluoroethylene; starches; gelatin; natural and synthetic gums; any other natural or synthetic polymer or fiber; and combinations thereof.

The backing layer can be any suitable material that is impermeable to the contents of the reservoir compartment, the polymer matrix, or the adhesive matrix. Suitable materials for backing films are well known to those skilled in the art and include, but are not limited to, occlusive polymers such as polyurethane, polyesters such as poly(ethylene phthalate), polyether amide, copolyester, polyisobutylene, polyesters, high and low density polyethylene, polypropylene, polyvinylchloride, metal foils, and metal foil laminates of suitable polymer films.

Suitable materials for the polymer matrix are well known to those skilled in the art and include, but are not limited to, polyethylene; polypropylene; ethylene/propylene copolymers; ethylene/ethylacrylate copolymers; ethylene/vinyl acetate copolymers; silicone elastomers, especially the medical-grade polydimethylsiloxanes; neoprene rubber; polyisobutylene; chlorinated polyethylene; polyvinyl chloride; vinyl chloride-vinyl acetate copolymer; polymethacrylate polymer (hydrogel); polyvinylidene chloride; poly(ethylene terephthalate); butyl rubber; epichlorohydrin rubbers; ethylene-vinyl alcohol copolymer; ethylene-vinyloxyethanol copolymer; silicone copolymers, for example, polysiloxane-polycarbonate copolymers, polysiloxane-polyethyleneoxide copolymers, polysiloxane-polymethacrylate copolymers, polysiloxane-alkylene copolymers (e.g., polysiloxane-ethylene copolymers), polysiloxane-alkylenesilane copolymers (e.g., polysiloxaneethylenesilane copolymers), and the like; cellulose polymers, for example methyl or ethyl cellulose, hydroxypropyl methyl cellulose, and cellulose esters; polycarbonates; polytetrafluoroethylene; and combinations thereof. In one embodiment, the polymer matrix has a glass transition temperatures below room temperature. The polymer can, but need not necessarily, have a degree of crystallinity at room temperature. Cross-linking monomeric units or sites can be incorporated into the polymers. For example, cross-linking monomers can be incorporated into polyacrylate polymers. The cross-linking monomers provide sites for cross-linking the polymer matrix after microdispersing the opioid, or a pharmaceutically acceptable salt thereof, and acyl opioid antagonist, or a pharmaceutically acceptable salt thereof, into the polymer. Known cross-linking monomers for polyacrylate polymers include, but are not limited to, polymethacrylic esters of polyols such as butylene diacrylate and dimethacrylate, trimethylol propane trimethacrylate, and the like. Other monomers that provide cross-linking sites include allyl acrylate, allyl methacrylate, diallyl maleate, and the like. In one embodiment, the polymer matrix does not allow any, or any detectable amount, of the acyl opioid antagonist, or a pharmaceutically acceptable salt thereof, to diffuse out of it, particularly in those instances in which the acyl opioid antagonist, or a pharmaceutically acceptable salt thereof, can penetrate a patient's skin.

Suitable materials for the pressure-sensitive adhesive matrix are well known to those skilled in the art and include, but are not limited to, polyisobutylenes, polysiloxanes, and polyacrylate copolymers (polyacrylic esters), natural rubber/karaya gum-based adhesives, hydrogels, hydrophilic polymers, and polyurethanes such as those described in H. Tan and W. Pfister, "Pressure Sensitive Adhesives for Transdermal Drug Delivery Systems," PSTT, 2 (February 1999). The adhesive may further include modifying monomers, tackifiers, plasticizers, fillers, waxes, oils, and other additives to impart the desired adhesive properties. *Id.* In one embodiment, the pressure-sensitive adhesive matrix that does not allow any, or any detectable amount, of the acyl opioid antagonist, or a pharmaceutically acceptable salt thereof, to diffuse out it, particularly in those instances in which the acyl opioid antagonist, or a pharmaceutically acceptable salt thereof, can penetrate a patient's skin.

Generally, the size of the device of can vary from about 1 cm² to greater than 200 cm² and typically are between about 5-50 cm². Methods for manufacturing transdermal-delivery devices are well known to those skilled in the art.

Examples of devices useful in the transdermal-delivery devices and methods of the invention include, but are not limited to, those described in U.S. Patent Nos. 4,806,341; 5,069,909; 5,236,714; 5,902,603; 5,914,718; and 6,162,456.

The transdermal-delivery device can optionally include one or more penetration enhancers, which increase the rate at which the opioid, or a pharmaceutically acceptable salt thereof, penetrates through the patient's skin. In one embodiment, the penetration enhancer does not enhance the penetration of the acyl opioid antagonist, or a pharmaceutically acceptable salt thereof, through the skin. In another embodiment, the penetration enhancer penetrates the rate-controlling membrane or diffuse out of the polymer matrix or adhesive matrix so that it can contact the patient's skin and improve penetration of the opioid, or a pharmaceutically acceptable salt thereof, through the patient's skin. Suitable penetration enhancers for use in the transdermal-delivery devices and methods of the invention include, but are not limited, C₂-C₄ alcohols such as ethanol and isopropanol, polyethylene glycol monolaurate, polyethylene glycol-3-lauramide, dimethyl lauramide, sorbitan trioleate, fatty acids, esters of fatty acids having from about 10 to about 20 carbon atoms, monoglycerides or mixtures of monoglycerides of fatty acids having a total monoesters content of at least 51% where the monoesters are those with from 10 to 20 carbon atoms, and mixtures of mono-,di- and tri-glycerides of fatty acids. Suitable fatty acids include, but are not limited to lauric acid, myristic acid, stearic acid, oleic acid, linoleic acid and palmitic acid. Monoglyceride permeation enhancers include glycerol monooleate, glycerol monolaurate, and glycerol monolinoleate, for example. Examples of penetration enhancers useful in the methods of the invention include, but are not limited to those described in U.S. Patent Nos. 3,472,931; 3,527,864; 3,896,238; 3,903,256; 3,952,099; 3,989,816; 4,046,886; 4,130,643; 4,130,667; 4,299,826; 4,335,115; 4,343,798; 4,379,454; 4,405,616; 4,746,515; 4,316,893; 4,405,616; 4,060,084, 4,379,454; 4,560,5534,863,952; 4,863,970; 4,879,275; 4,940,586; 4,960,771; 4,973,968; 5,066,648; 5,164,406; 5,227,169; 5,229,130; 5,238,933; 5,308,625; 4,326,566; 5,378,730; 5,420,106; 5,641,5045,716,638; 5,750,137;5,785,991; 5,837,289; 5,834,468; 5,882,676; 5,912,009; 5,952,000; 6,004,578; and Idson, Percutaneous Absorption, J. Pharm. Sci. 64(b6):901-924 ( June 1975).

The transdermal-delivery device can further comprise an other additive conventionally used in therapeutic products. For example, the transdermal-delivery device can also include one or more preservatives or bacteriostatic agents, *e.g.*, methyl hydroxybenzoate, propyl hydroxybenzoate, chlorocresol, benzalkonium chlorides, and the like; or other active ingredients such as antimicrobial agents, particularly antibiotics; anesthetics; other analgesics; and antipruritic agents.

### OPIOIDS

Any opioid, or a pharmaceutically acceptable salt thereof, can be used in the transdermal-delivery device of the present invention. Opioids include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dihydromorphone, dihydroisomorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl, heroin, hydrocodone, hydromorphone, hydromorphodone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, pantopon, papaveretum, paregoric, pentazocine, phenadoxone, phendimetrazine, phendimetrazone, phenomorphan, phenazocine, phenoperidine, piminodine, piritramide, propheptazine, promedol, properidine, propoxyphene, propylhexedrine, sufentanil, tilidine, tramadol, and mixtures thereof.

In certain embodiments, the opioid agonist is hydrocodone, morphine, hydromorphone, oxycodone, codeine, levorphanol, meperidine, methadone, oxymorphone, buprenorphine, fentanyl, dipipanone, heroin, tramadol, etorphine, dihydroetorphine, butorphanol, levorphanol, pharmaceutically acceptable salts thereof, and mixtures thereof. In one embodiment ,the opioid agonist is oxycodone, hydrocodone, fentanyl, buprenorphine, or a pharmaceutically acceptable salt thereof.

In one embodiment, especially for passive patches, the opioid is a free-base opioid, i.e., not a pharmaceutically acceptable salt of the opioid. However, for patches that use iontophoresis to facilitate penetration of the skin by the opioid, or a pharmaceutically acceptable salt thereof, the pharmaceutically acceptable salt of the opioid is preferred.

The analgesically effective amount of opioid, or a pharmaceutically acceptable salt thereof, present in the transdermal-delivery device will depend on the specific opioid, or a pharmaceutically acceptable salt thereof, the type of device, the materials used to manufacture the device, and the duration for which the opioid, or a pharmaceutically acceptable salt thereof, will be delivered to the patient. The analgesically effective amount of opioid, or a pharmaceutically acceptable salt thereof, present in the transdermal-delivery device, however, is typically from about 0.1 to about 500 mg, in one embodiment, from about 1 to about 100 mg; and in another embodiment from about 1 to about 50 mg. It is well within the purview of one skilled in the art to readily determine the analgesically effective amount of opioid, or a pharmaceutically acceptable salt thereof, needed for a particular indication.

### THE ACYL OPIOID ANTAGONIST

The acyl opioid antagonist has the formula: 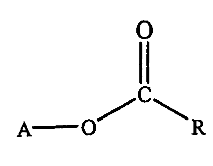 wherein "A-O-" is a portion of the opioid antagonist having one or more hydroxyl groups, less a proton (H⁺) of one of the hydroxyl groups; and "R-C(O)" is an acyl group.

Opioid antagonists that can be used in the transdermal-delivery devices and methods of the invention include, but are not limited to, cyclazocine, naloxone, naltrexone, nalmefene, nalbuphine, nalorphine, cyclazacine, levallorphan, pharmaceutically acceptable salts thereof, and mixtures thereof. In this regard, A-O-C(O)-R is an acyl derivative of cyclazocine, naloxone, naltrexone, nalmefene, nalbuphine, nalorphine, cyclazacine, levallorphan, or a pharmaceutically acceptable salt thereof. In certain embodiments, the opioid antagonist is nalmefene, naloxone, naltrexone, or a pharmaceutically acceptable salt thereof. In one embodiment, a pharmaceutically acceptable salt of an acyl opioid antagonist is used in the transdermal-delivery device of the invention, especially when the transdermal-delivery device is a passive device.

Representative R-C(O) groups include, but are not limited to, (C₁-C₆)-C(O), such as acetyl and propionyl; phenylacetyl; tartaryl; glutamyl, succinyl, benzoyl that is unsubstituted or substituted with one or more C₁-C₃ alkyl, -CF₃, -NO₂, -halogen, or -O(C₁ - C₃ alkyl) groups; 4-hydroxybutyryl; glycolyl; lactyl; aspartyl; sulfanilyl; citryl; fumaryl; pamoyl; malyl; maleyl; malonyl; hydroxymaleyl; p-toluenesulfonyl; steraryl; glucuronyl; HOC(O)-R'-C(O), wherein R' is a C₃ - C₄ alkyl group, optionally substituted with a C₁ - C₄ alkyl group; C₈-C₁₆ alkanoyl, such as octanoyl, nonanoyl, decanoyl, undecanoyl, dodecanoyl, tetradecanoyl, and hexadecanoyl; C₈-C₁₈ alkenoyl such as palmitoyl, oleoyl, linoleoyl, and linolenoyl; and 2-aminoacyl. In one embodiment, R-C(O) is benzoyl, p-nitrobenzoyl, p-anisoyl, p-toluyl, or acetyl.

In one embodiment, the acyl opioid antagonist is a 14-(acetyl)nalmefine, 3-(acetyl)nalbuphine, 6-(acetyl)nalbuphine, or 14-(acetyl)nabuphine, or a pharmaceutically acceptable salt thereof.

In another embodiment, the acyl opioid antagonist is 8-(phenylacetyl)cyclazocine, 3-(phenylacetyl)naloxone, 14-(phenylacety)lnaloxone, 3-(phenylacetyl)naltrexone, 14-(phenylacetyl)naltrexone, 3-(phenylacetyl)levallorphan, 3-(phenylacetyl)nalmefene, 14-(phenylacetyl)nalmefene, 3-(phenylacetyl)nalbuphine, 6-(phenylacetyl)nalbuphine, 14-(phenylacetyl)nalbuphine, 6-(phenylacetyl)nalorphine, 3-(phenylacetyl)nalorphine, or a pharmaceutically acceptable salt thereof.

In another embodiment the acyl opioid antagonist is 8-(tartaryl)cyclazocine, 3-(tartaryl)naloxone, 14-(tartaryl)naloxone, 3-(tartaryl)naltrexone, 14-(tartaryl)naltrexone, 3-(tartaryl)levallorphan, 3-(tartaryl)nalmefene, 14-(tartaryl)nalmefene, 3-(tartaryl)nalbuphine, 6-(tartaryl)nalbuphine, 14-(tartaryl)nalbuphine, 6-(tartaryl)nalorphine, 3-(tartaryl)nalorphine, or a pharmaceutically acceptable salt thereof.

In another embodiment the acyl opioid antagonist is 8-(propionyl)cyclazocine, 3-(propionyl)naloxone, 14-(propionyl)naloxone, 3-(propionyl)naltrexone, 14-(propionyl)naltrexone, 3-(propionyl)nalmefene, 14-(propionyl)nalmefene, 6-(propionyl)nalbuphine, 14-(propionyl)nalbuphine, 6-(propionyl)nalorphine, 3-(propionyl)nalorphine, or a pharmaceutically acceptable salt thereof.

In another embodiment the acyl opioid antagonist is 8-(1-glutamyl)cyclazocine, 3-(1-glutamyl)naloxone, 14-(1-glutamyl)naloxone, 3-(1-glutamyl)naltrexone, 14-(1-glutamyl)naltrexone, 3-(1-glutamyl)levallorphan, 3-(1-glutamyl)nalmefene, 14-(1-glutamyl)nalmefene, 3-(1-glutamyl)nalbuphine, 6-(1-glutamyl)nalbuphine, 14-(1-glutamyl)nalbuphine, 6-(1-glutamyl)nalorphine, 3-(1-glutamyl)nalorphine, or a pharmaceutically acceptable salt thereof.

In another embodiment the acyl opioid antagonist is 8-(5-glutamyl)cyclazocine, 3-(5-glutamyl)naloxone, 14-(5-glutamyl)naloxone, 3-(5-glutamyl)naltrexone, 14-(5-glutamyl)naltrexone, 3-(5-glutamyl)levallorphan, 3-(5-glutamyl)nalmefene, 14-(5-glutamyl)nalmefene, 3-(5-glutamyl)nalbuphine, 6-(5-glutamyl)nalbuphine, 14-(5-glutamyl)nalbuphine, 6-(5-glutamyl)nalorphine, 3-(5-glutamyl)nalorphine, or a pharmaceutically acceptable salt thereof.

In another embodiment the acyl opioid antagonist is a benzoyl ester, optionally substituted with a C₁-C₃ alkyl group, -NO₂, -halogen, -OH, or -O(C₁ - C₃ alkyl), formed with the 8-hydroxyl group of cyclazocine, the 3- or 14-hydroxyl group of naloxone, the 14-hydroxyl group of naltrexone, the 3-hydroxyl group of levallorphan, the 3- or 14-hydroxyl group of nalmefene, the 6- or 14-hydroxyl group of nalbuphine, or the 3- or 6-hydroxyl group of nalorphine, or a pharmaceutically acceptable salt thereof.

In another embodiment the acyl opioid antagonist is 8-(succinyl)cyclazocine, 3-(succinyl)naloxone, 14-(succinyl)naloxone, 3-(succinyl)naltrexone, 14-(succinyl)naltrexone, 3-(succinyl)levallorphan, 3-(succinyl)nalmefene, 14-(succinyl)nalmefene, 3-(succinyl)nalbuphine, 6-(succinyl)nalbuphine, 14-(succinyl)nalbuphine, 6-(succinyl)nalorphine, 3-(succinyl)nalorphine, or a pharmaceutically acceptable salt thereof.

In another embodiment the acyl opioid antagonist is 8-(4-hydroxybutyryl)cyclazocine, 3-(4-hydroxybutyryl)naloxone, 14-(4-hydroxybutyryl)naloxone, 3-(4-hydroxybutyryl)naltrexone, 14-(4-hydroxybutyryl)naltrexone, 3-(4-hydroxybutyryl)levallorphan, 3-(4-hydroxybutyryl)nalmefene, 14-(4-hydroxybutyryl)nalmefene, 3-(4-hydroxybutyryl)nalbuphine, 6-(4-hydroxybutyryl)nalbuphine, 14-(4-hydroxybutyryl)nalbuphine, 6-(4-hydroxybutyryl)nalorphine, 3-(4-hydroxybutyryl)nalorphine, or a pharmaceutically acceptable salt thereof.

In another embodiment the acyl opioid antagonist is 8-(glycolyl)cyclazocine, 3-(glycolyl)naloxone, 14-(glycolyl)naloxone, 3-(glycolyl)naltrexone, 14-(glycolyl)naltrexone, 3-(glycolyl)levallorphan, 3-(glycolyl)nalmefene, 14-(glycolyl)nalmefene, 3-(glycolyl)nalbuphine, 6-(glycolyl)nalbuphine, 14-(glycolyl)nalbuphine, 6-(glycolyl)nalorphine, 3-(glycolyl)nalorphine, or a pharmaceutically acceptable salt thereof.

In another embodiment the acyl opioid antagonist is 8-(lactyl)cyclazocine, 3-(lactyl)naloxone, 14-(lactyl)naloxone, 3-(lactyl)naltrexone, 14-(lactyl)naltrexone, 3-(lactyl)levallorphan, 3-(lactyl)nalmefene, 14-(lactyl)nalmefene, 3-(lactyl)nalbuphine, 6-(lactyl)nalbuphine, 14-(lactyl)nalbuphine, 6-(lactyl)nalorphine, 3-(lactyl)nalorphine, or a pharmaceutically acceptable salt thereof.

In another embodiment the acyl opioid antagonist is 8-(1-aspartyl)cyclazocine, 3-(1-aspartyl)naloxone, 14-(1-aspartyl)naloxone, 3-(1-aspartyl)naltrexone, 14-(1-aspartyl)naltrexone, 3-(1-aspartyl)levallorphan, 3-(1-aspartyl)nalmefene, 14-(1-aspartyl)nalmefene, 3-(1-aspartyl)nalbuphine, 6-(1-aspartyl)nalbuphine, 14-(1-aspartyl)nalbuphine, 6-(1-aspartyl)nalorphine, 3-(1-aspartyl)nalorphine, or a pharmaceutically acceptable salt thereof.

In another embodiment the acyl opioid antagonist is 8-(4-aspartyl)cyclazocine, 3-(4-aspartyl)naloxone, 14-(4-aspartyl)naloxone, 3-(4-aspartyl)naltrexone, 14-(4-aspartyl)naltrexone, 3-(4-aspartyl)levallorphan, 3-(4-aspartyl)nalmefene, 14-(4-aspartyl)nalmefene, 3-(4-aspartyl)nalbuphine, 6-(4-asparlyl)nalbuphine, 14-(4-aspartyl)nalbuphine, 6-(4-aspartyl)nalorphine, 3-(4-aspartyl)nalorphine, or a pharmaceutically acceptable salt thereof.

In another embodiment the acyl opioid antagonist is 8-(sulfanilyl)cyclazocine, 3-(sulfanilyl)naloxone, 14-(sulfanilyl)naloxone, 3-(sulfanilyl)naltrexone, 14-(sulfanilyl)naltrexone, 3-(sulfanilyl)levallorphan, 3-(sulfanilyl)nalmefene, 14-(sulfanilyl)nalmefene, 3-(sulfanilyl)nalbuphine, 6-(sulfanilyl)nalbuphine, 14-(sulfanilyl)nalbuphine, 6-(sulfanilyl)nalorphine, 3-(sulfanilyl)nalorphine, or a pharmaceutically acceptable salt thereof.

In another embodiment the acyl opioid antagonist is 8-(fumaryl)cyclazocine, 3-(fumaryl)naloxone, 14-(fumaryl)naloxone, 3-(fumaryl)naltrexone, 14-(fumaryl)naltrexone, 3-(fumaryl)levallorphan, 3-(fumaryl)nalmefene, 14-(fumaryl)nalmefene, 3-(fumaryl)nalbuphine, 6-(fumaryl)nalbuphine, 14-(fumaryl)nalbuphine, 6-(fumaryl)nalorphine, 3-(fumaryl)nalorphine, or a pharmaceutically acceptable salt thereof.

In another embodiment the acyl opioid antagonist is 8-(pamoyl)cyclazocine, 3-(pamoyl)naloxone, 14-(pamoyl)naloxone, 3-(pamoyl)naltrexone, 14-(pamoyl)naltrexone, 3-(pamoyl)levallorphan, 3-(pamoyl)nalmefene, 14-(pamoyl)nalmefene, 3-(pamoyl)nalbuphine, 6-(pamoyl)nalbuphine, 14-(pamoyl)nalbuphine, 6-(pamoyl)nalorphine, 3-(pamoyl)nalorphine, or a pharmaceutically acceptable salt thereof.

In another embodiment the acyl opioid antagonist is 8-(1-malyl)cyclazocine, 3-(1-malyl)naloxone, 14-(1-malyl)naloxone, 3-(1-malyl)naltrexone, 14-(1-malyl)naltrexone, 3-(1-malyl)levallorphan, 3-(1-malyl)nalmefene, 14-(1-malyl)nalmefene, 3-(1-malyl)nalbuphine, 6-(1-malyl)nalbuphine, 14-(1-malyl)nalbuphine, 6-(1-malyl)nalorphine, 3-(1-malyl)nalorphine, or a pharmaceutically acceptable salt thereof.

In another embodiment the acyl opioid antagonist is 8-(4-malyl)cyclazocine, 3-(4-malyl)naloxone, 14-(4-malyl)naloxone, 3-(4-malyl)naltrexone, 14-(4-malyl)naltrexone, 3-(4-malyl)levallorphan, 3-(4-malyl)nalinefene, 14-(4-malyl)nalmefene, 3-(4-malyl)nalbuphine, 6-(4-malyl)nalbuphine, 14-(4-malyl)nalbuphine, 6-(4-malyl)nalorphine, 3-(4-malyl)nalorphine, or a pharmaceutically acceptable salt thereof.

In another embodiment the acyl opioid antagonist is 8-(maleyl)cyclazocine, 3-(maleyl)naloxone, 14-(maleyl)naloxone, 3-(maleyl)naltrexone, 14-(maleyl)naltrexone, 3-(maleyl)levallorphan, 3-(maleyl)nalmefene, 14-(maleyl)nalinefene, 3-(maleyl)nalbuphine, 6-(maleyl)nalbuphine, 14-(maleyl)nalbuphine, 6-(maleyl)nalorphine, 3-(maleyl)nalorphine, or a pharmaceutically acceptable salt thereof.

In another embodiment the acyl opioid antagonist is 8-(1-(2-hydroxy)maleyl)cyclazocine, 3-(1-(2-hydroxy)maleyl)naloxone, 14-(1-(2-hydroxy)maleyl)naloxone, 3-(1-(2-hydroxy)maleyl)naltrexone, 14-(1-(2-hydroxy)maleyl)naltrexone, 3-(1-(2-hydroxy)maleyl)levallorphan, 3-(1-(2-hydroxy)maleyl)nalmefene, 14-(1-(2-hydroxy)maleyl)nalmefene, 3-(1-(2-hydroxy)maleyl)nalbuphine, 6-(1-(2-hydroxy)maleyl)nalbuphine, 14-(1-(2-hydroxy)maleyl)nalbuphine, 6-(1-(2-hydroxy)maleyl)nalorphine, 3-(1-(2-hydroxy)maleyl)nalorphine, or a pharmaceutically acceptable salt thereof.

In another embodiment the acyl opioid antagonist is 8-(4-(2-hydroxy)maleyl)cyclazocine, 3-(4-(2-hydroxy)maleyl)naloxone, 14-(4-(2-hydroxy)maleyl)naloxone, 3-(4-(2-hydroxy)maleyl)naltrexone, 14-(4-(2-hydroxy)maleyl)naltrexone, 3-(4-(2-hydroxy)maleyl)levallorphan, 3-(4-(2-hydroxy)maleyl)nalmefene, 14-(4-(2-hydroxy)maleyl)nalmefene, 3-(4-(2-hydroxy)maleyl)nalbuphine, 6-(4-(2-hydroxy)maleyl)nalbuphine, 14-(4-(2-hydroxy)maleyl)nalbuphine, 6-(4-(2-hydroxy)maleyl)nalorphine, 3-(4-(2-hydroxy)maleyl)nalorphine, or a pharmaceutically acceptable salt thereof.

In another embodiment the acyl opioid antagonist is 8-(p-toluenesulfonyl)cyclazocine, 3-(p-toluenesulfonyl)naloxone, 14-(p-toluenesulfonyl)naloxone, 3-(p-toluenesulfonyl)naltrexone, 14-(p-toluenesulfonyl)naltrexone, 3-(p-toluenesulfonyl)levallorphan, 3-(p-toluenesulfonyl)nalmefene, 14-(p-toluenesulfonyl)nalmefene, 3-(p-toluenesulfonyl)nalbuphine, 6-(p-toluenesulfonyl)nalbuphine, 14-(p-toluenesulfonyl)nalbuphine, 6-(p-toluenesulfonyl)nalorphine, 3-(p-toluenesulfonyl)nalorphine, or a pharmaceutically acceptable salt thereof.

In another embodiment the acyl opioid antagonist is 8-(stearyl)cyclazocine, 3-(stearyl)naloxone, 14-(stearyl)naloxone, 3-(stearyl)naltrexone, 14-(stearyl)naltrexone, 3-(stearyl)levallorphan, 3-(stearyl)nalmefene, 14-(stearyl)nalmefene, 3-(stearyl)nalbuphine, 6-(stearyl)nalbuphine, 14-(stearyl)nalbuphine, 6-(stearyl)nalorphine, 3-(stearyl)nalorphine, or a pharmaceutically acceptable salt thereof.

In another embodiment the acyl opioid antagonist is 3-(malonyl)naloxone, 14-(malonyl)naloxone, 3-(malonyl)naltrexone, 14-(malonyl)naltrexone, 3-(malonyl)nalmefine, 14-(malonyl)nalmefine, 3-(malonyl)nalbuphine, 6-(malonyl)nalbuphine, 14-malonyl)nalbuphine, 3-(malonyl)naloorphine, 6-(malonyl)-nalorphine, 8-(malonyl)-cyclazocine, 3-(malonyl)-levallorphan, or a pharmaceutically acceptable salt thereof.

In another embodiment the acyl opioid antagonist is 3-(glucuronyl)naloxone, 14-(glucuronyl)naloxone, 3-(glucuronyl)naltrexone, 14-(glucuronyl)naltrexone, 3-(glucuronyl)nalmefine, 14-(glucuronyl)nalmefine, 3-(glucuronyl)nalbuphine, 6-(glucuronyl)nalbuphine, 14-glucuronyl)nalbuphine, 3-(glucuronyl)naloorphine, 6-(glucuronyl)-nalorphine, 8-(glucuronyl)-cyclazocine, 3-(glucuronyl)-levallorphan, or a pharmaceutically acceptable salt thereof.

In another embodiment the acyl opioid antagonist is an ester of an α-amino acid formed with the 8-hydroxyl group of cyclazocine, the 3- or 14-hydroxyl group of naloxone, the 3- or 14-hydroxyl group of naltrexone, the 3-hydroxyl group of levallorphan, the 3- or 14-hydroxyl group of nalmefene, the 3-, 6-, or 14-hydroxyl group ofnalbuphine or the 3- or 6-hydroxyl group of nalorphine, or a pharmaceutically acceptable salt thereof.

In another embodiment the acyl opioid antagonist is a monoester of a C₅ - C₆ dicarboxylic acid, formed with the 8-hydroxyl group of cyclazocine, the 3- or 14-hydroxyl group of naloxone, the 3- or 14-hydroxyl group of naltrexone, the 3-hydroxyl group of levallorphan, the 3- or 14-hydroxyl group of nalmefene, the 3-, 6-, or 14-hydroxyl group of nalbuphine, or the 3- or 6-hydroxyl group of nalorphine, or a pharmaceutically acceptable salt thereof.

In another embodiment, the acyl opioid antagonist is 3-(1-(2, 2-dimethylsuccinyl))naltrexone, 3-(4-(2, 2-dimethylsuccinyl))naltrexone, 14-(1-(2, 2-dimethylsuccinyl))naltrexone, 14-(4-(2,2-dimethylsuccinyl))naltrexone, 3-(3, 3-dimethylglutamyl)naltrexone, 14-(3, 3-dimethylglutamyl)naltrexone, or a pharmaceutically acceptable salt thereof.

Without wishing to be bound by theory it is believed that in certain embodiments the acyl opioid antagonist, or a pharmaceutically acceptable salt thereof, does not penetrate the skin or does so in an amount insufficient to inhibit the analgesic effect of the opioid, or a pharmaceutically acceptable salt thereof, because of the physico-chemical properties or increased size of the acyl opioid antagonist, or a pharmaceutically acceptable salt thereof, relative to the opioid antagonist, or a pharmaceutically acceptable salt thereof. Also, and without wishing to be bound by theory it is believed that in certain embodiments the acyl opioid antagonist, or a pharmaceutically acceptable salt thereof, does not penetrate the rate-controlling membrane of a transdermal-delivery device or does so in an amount insufficient to inhibit the analgesic effect of the opioid, or a pharmaceutically acceptable salt thereof, because of the physico-chemical properties or increased size of the acyl opioid antagonist, or a pharmaceutically acceptable salt thereof, relative to the opioid antagonist, or a pharmaceutically acceptable salt thereof. In addition, and without wishing to be bound by theory, it is believed that in certain embodiments the acyl opioid antagonist, or a pharmaceutically acceptable salt thereof, does not diffuse out of the polymer matrix or adhesive matrix of a transdermal-delivery device or does so in an amount insufficient to inhibit the analgesic effect of the opioid antagonist, or a pharmaceutically acceptable salt thereof, because the ester is highly soluble in or has a high affinity for the polymer matrix or adhesive matrix. One skilled in the art knows how to select a polymer matrix or adhesive matrix for which a given acyl opioid antagonist, or a pharmaceutically acceptable salt thereof, will not diffuse out of it. Without being bound by theory, Applicants also believe that the increased molecular weight of the opioid antagonist, or a pharmaceutically acceptable salt thereof, relative to the opioid antagonist, or a pharmaceutically acceptable salt thereof, limits the ability of the acyl opioid antagonist, or a pharmaceutically acceptable salt thereof, to penetrate the skin.

In one embodiment, the acyl opioid antagonist comprises the opioid antagonist and the functionality of a penetration enhancer. In this embodiment, R-C(O)OH is a penetration enhancer, and R-C(O) is the penetration enhancer less the -OH group of one of the penetration enhancer's carboxyl groups. Examples of penetration enhancers having carboxyl groups include, but are not limited to, saturated fatty acids such as hexanoic acid, heptanoic acid, octanoic acid, nonanoic acid, decanoic acid, undecanoic acid, myristic acid, and lauric acid; monounsaturated fatty acids such as cis-octadecanoic (petroselinic) acid, cis-9-octadecanoic (oleic) acid, cis-11-octadecanoic (vaccenic) acid, and cis-13-octadecanoic acid; and polyunsaturated fatty acids such as linoleic acid (all cis 9, 12-octadecanoic acid), linolenic acid (all cis 9, 12, 15-octadecanoic acid) and arachidonioc acid (all cis 5, 8, 11, 14-eicosatetraenoic acid) (*See, e.g.,* H. Tanjo and H.E. Junginger, Skin Permeation Enhancement by Fatty Acids, J. Dispersion Science and Technology, 20(1&2):127-138 (1999) and U.S. Patent No. 4,626,539.

Without wishing to be bound by theory, it is also believed that when the acyl opioid antagonist, or a pharmaceutically acceptable salt thereof, is hydrolyzed, for example, as a result of a person attempting to extract or abuse the opioid component of the transdermal delivery-device, it provides the opioid antagonist, or a pharmaceutically acceptable salt thereof, and a penetration enhancer that will increase penetration of the opioid antagonist, or a pharmaceutically acceptable salt thereof, and enhance its ability to inhibit the euphoric effect of the opioid, or pharmaceutically acceptable salt thereof.

In another embodiment the transdermal-delivery device is a polymer-matrix transdermal-delivery device or a drug-in-adhesive transdermal-delivery device, and the A-O- portion of the acyl opiod antagonist forms an acyl linkage with a carboxylic acid functional group of the polymer matrix or the adhesive matrix. In this case, the polymer matrix or the adhesive matrix is typically a polyacrylate matrix. In one embodiment, polyacrylic acid or polyacrylate monomers are copolymerized with an acyl opioid antagonist, or a pharmaceutically acceptable salt thereof, wherein the acyl group of the acyl opioid antagonist is an acryloyl group *(See, e.g.,* H.S. Tan and W R. Pfister, *Pressure Sensitive Adhesives for Transdermal Drug Delivery Systems,* PSTT, 2,(2):60-69 (Feb. 1999).

In another embodiment, R-C(O)OH is a nutritive acid, and R-C(O)O is the nutritive acid less the -OH group of one of the nutritive acid's carboxyl groups. Example of nutritive acids having one or more carboxyl groups include, but are not limited to, amino acids; fatty acids; or an aldonic acid, *i.e.,* an aldose, whose formyl group is replaced with a carboxylic acid group.

The amount of the acyl opioid antagonist, or a pharmaceutically acceptable salt thereof, present in the transdermal-delivery device and sufficient to inhibit the euphoric effect of the opioid, of pharmaceutically acceptable salt thereof, will depend on the specific acyl opioid antagonist, or a pharmaceutically acceptable salt thereof. Typically, the molar ratio of the acyl opioid antagonist, or a pharmaceutically acceptable salt thereof, to the opioid, or a pharmaceutically acceptable salt thereof, in the transdermal-delivery device ranges from about 1:16 to about 3:1; in one embodiment from about 1:10 to about 2:1; in another embodiment from about 1:5 to about 1:1; and in another embodiment from about 1:3 to about 1:1. One skilled in the art can readily determine the amount of acyl opioid antagonist, or a pharmaceutically acceptable salt thereof, needed for a particular indication.

### SYNTHESIS OF THE ACYL OPIOID ANTOGONIST

The acyl opioid antagonists can be obtained using conventional organic syntheses or by the following illustrative methods shown in the scheme below: 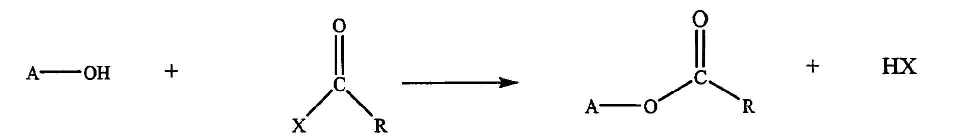 wherein:
A-OH is an opioid antagonist,
X is a leaving group, and
R-C(O) is defined above.

The acyl opioid antagonist, or a pharmaceutically acceptable salt thereof, can be obtained by esterifying the -OH (hydroxyl) group of the opioid antagonist, or a pharmaceutically acceptable salt thereof. A-OH can be esterified via reaction with an acid halide (where X = halide) in the presence of a base. In one embodiment, the acid halide is an acid chloride. The reaction can be run in an aqueous or non-aqueous solvent. Suitable non-aqueous solvents include, but are not limited to, diethyl ether, tetrahydrofuran, acetonitrile, chloroform, carbon tetrachloride, and dichloromethane. The base can be an inorganic base such as NaHCO₃ or an organic amine. In one embodiment the organic amine is a tertiary organic amine. In another embodiment, the organic amine is pyridine or triethylamine. Methods for forming esters from alcohols or phenols and acid halides are well known to those skilled in the art and are described in J. March, Advanced Organic Chemistry, Reaction Mechanisms and Structure, 4th ed. John Wiley & Sons, NY, 1992, p. 392.

The A-OH can also be esterified via reaction with an acid anhydride (where X = R-C(O)O). This reaction is typically run in an aprotic solvent, such as one of those described above, in the presence of a protic acid, Lewis acid, or base catalyst. In one embodiment, the catalyst is an organic amine. In one embodiment, the organic amine is pyridine or 4-(N, N-dimethylamino)pyridine. A suitable non-basic catalyst is cobalt (II) chloride. Methods for forming esters from alcohols or phenols and acid anhydrides are well known to those skilled in the art and are described in J. March, Advanced Organic Chemistry, Reaction Mechanisms and Structure, 4th ed. John Wiley & Sons, NY, 1992, pp. 392-393.

The A-OH can also be esterified via reaction with a carboxylic acid in an aprotic solvent, such as those described above, in the presence of a dehydrating agent. Suitable dehydrating agents include, but are not limited to dicyclohexylcarbodimide, an alkyl chloroformate and triethylamine, pyridinium salts-Bu₃N, phenyl dichlorophosphate (PhOPOCl₂), dicyclohexylcarbodimide and an aminopyridine, 2-chloro-1, 3, 5-trinitrobenzene and pyridine, di-2-pyridyl carbonate, polystyryl diphenylphosphine, (trimethylsilyl)ethoxyacetylene, 1, 1'-carbonylbis(3-methylimidazolium) triflate, amberlyst-157, diethyl azodicarboxylate (EtOOCN=NCOOEt) and Ph₃P, chlorosulfonyl isocyanate (CISO₂NCO), chlorosilanes, MeSO₂Cl-Et₃N, Ph₃P-CCl₄-Et₃N, and N, N'-carbonyldiimidazole. Methods for forming esters from an alcohol or phenol and a carboxylic acid are well known to those skilled in the art and are described in J. March, Advanced Organic Chemistry, Reaction Mechanisms and Structure, 4th ed. John Wiley & Sons, NY, 1992, pp. 393-396.

Once formed, the acyl opioid antagonist, or a pharmaceutically acceptable salt thereof, can be isolated from a chemical reaction mixture by partitioning the reaction products between an organic phase and an aqueous phase, drying the organic phase (for example, with anhydrous magnesium sulfate or anhydrous sodium sulfate), and concentrating it, typically under reduced pressure, to provide the acyl opioid antagonist, or a pharmaceutically acceptable salt thereof. The acyl opioid antagonist, or a pharmaceutically acceptable salt thereof, can then be further purified by conventional techniques well known to those skilled in the art including, but not limited to, column chromatography, high pressure liquid chromatography (HPLC), gas chromatography (GC), recrystallization, and/or distillation.

Methods for preparing the acid halides are well known to those skilled in the art and are described in J. March, Advanced Organic Chemistry, Reaction Mechanisms and Structure, 4th ed. John Wiley & Sons, NY, 1992, pp. 437-8. For example, acid halides can be prepared by reacting the carboxylic acid with thionyl chloride, bromide, or iodide. Acid chlorides and bromides can also be prepared by reacting a carboxylic acid with phosphorous trichloride or phosphorous tribromide, respectively. Acid chlorides can also be prepared by reacting a carboxylic acid with Ph₃P in carbon tetrachloride. Acid fluorides can be prepared by reacting a carboxylic acid with cyanuric fluoride.

Methods for preparing acid anhydrides are well-known to those skilled in the art and are described in J. March, Advanced Organic Chemistry, Reaction Mechanisms and Structure, 4th ed. John Wiley & Sons, NY, 1992, pp. 400-402. For example, acid anhydrides can be prepared by reacting an acid chloride with a carboxylic acid salt, typically a sodium, potassium, or silver salt, typically in an aprotic solvent. In one embodiment, the reaction is run in the presence of pyridine. Acid anhydrides can also be prepared by reacting two equivalents of a carboxylic acid, RCOOH, in an aprotic solvent in the presence of a dehydrating agent. Suitable dehydrating agents include, but are not limited to, acetic anhydride, trifluoroacetic anhydride, dicyclohexylcarbodiimide, methoxyacetylene, P₂O₅, trimethylsilylethoxyacetylene, Me₃SiC ≡COEt, and tetracyanoethylene and a base.

When the opioid antagonist, or a pharmaceutically acceptable salt thereof, has more than one hydroxyl group it is possible to selectively esterify one of the hydroxyl groups. If one of the hydroxyl groups is more reactive than the other, the more reactive hydroxyl group can be selectively esterified by reacting about 1 eq. of the opioid antagonist, or a pharmaceutically acceptable salt thereof, with about 1 eq. of an acid chloride or anhydride. For example, if one of the hydroxyl groups is a phenolic hydroxyl and the other hydroxyl is non-phenolic, the reactivity of the phenolic hydroxyl can be increased by deprotonating it to provide a more reactive phenoxide ion. The phenoxide ion is then selectively esterified using about 1 eq. of an acid chloride or acid anhydride. The phenolic hydroxyl can be easily deprotonated by reacting it with 1 equivalent of a base, such as lithium methoxide in methanol or sodium hydride.

The less reactive hydroxyl group can be selectively esterified by first reacting the more reactive hydroxyl group with a protecting group, esterifying the less reactive hydroxyl group, and then removing the protecting group. For example, if the opioid antagonist, or a pharmaceutically acceptable salt thereof, has a phenolic hydroxyl and a non-phenolic hydroxyl, the latter can be selectively esterified by first deprotonating the phenolic hydroxyl to provide a more reactive phenoxide ion; reacting the phenoxide ion with a protecting group to provide a protected opioid antagonist, or a pharmaceutically acceptable salt thereof; esterifying the remaining hydroxyl of the protected opioid antagonist, or a pharmaceutically acceptable salt thereof; and then removing the protecting group. Suitable protecting groups include, but are not limited to, a benzyl ether, trimethylsilyl ether, isopropyldimethylsilyl ether, t-butyldimethylsilyl ether, t-butyldiphenylsilyl ether, tribenzylsilyl ether, and triisopropylsilyl ether *(See, e.g.,* T.W. Greene, Protective Groups in Organic Synthesis, John Wiley-Interscience Publication, New York, (1981)).

Acyl opioid antagonist wherein R-C(O) is glucuronyl can be prepared by any known or later developed method for forming O-gluconoridic esters. Representative methods include, but are not limited to, those described in Bowering and Timell, J. Amer. Chem. Soc., 82: 2827, 2829 (1960); Goebel et al., J. Biol. Chem., 106:63, 65 (1934); Leu, et al., J. Med. Chem., 42:18, 3623-28 (1999), and Juteau et al., Tett. Lett. 38(9) 1481-4 (1997),.

### METHODS OF TREATING OR PREVENTING PAIN

In accordance with the invention, the transdermal-delivery device of the invention can be used to administer to a patient, in one embodiment a mammal, and in another embodiment a human, an analgesically effective amount of an opioid, or a pharmaceutically acceptable salt thereof, for the treatment or prevention of pain. The transdermal-delivery device can be used to treat or prevent acute or chronic pain. For example, the transdermal-delivery device can be used for, but is not limited to, treating or preventing cancer pain, central pain, labor pain, myocardial infarction pain, pancreatic pain, colic pain, post-operative pain, headache pain, muscle pain, bone pain, and pain associated with intensive care.

According to the methods of the invention the transdermal-delivery device is contacted with the skin of the patient, and the opioid, or a pharmaceutically acceptable salt thereof, is released by the transdermal-delivery device and becomes absorbed through the skin of the patient. Once absorbed, the opioid, or a pharmaceutically acceptable salt thereof, enters into the patient's circulatory system providing an analgesically effective amount of the opioid, or a pharmaceutically acceptable salt thereof. In one embodiment, the transdermal delivery device is contacted with a patient's skin for from about 12 h to about 2 weeks. In another embodiment, the transdermal delivery device is contacted with a patient's skin for from about 24 h to about 1 week. In another embodiment, the transdermal delivery device is contacted with a patient's skin for from about 3 days to about 1 week. The transdermal-delivery device can provide sustained and continuous delivery of an analgesically effective amount of the opioid, or a pharmaceutically acceptable salt thereof. In one embodiment, the transdermal-delivery device of the invention maintains a level of the opioid, or a pharmaceutically acceptable salt thereof, in the bloodstream of the patient between about 0.1 to about 100 nanograms of opioid, or a pharmaceutically acceptable salt thereof, per milliliter of blood plasma for about 16 hours to about 7 days, in one embodiment about 16 hours to about 72 hours, and in another embodiment at least about 24 hours.

### EXAMPLES

### Example 1: Synthesis of 3-(p-anisoylnaltrexone).

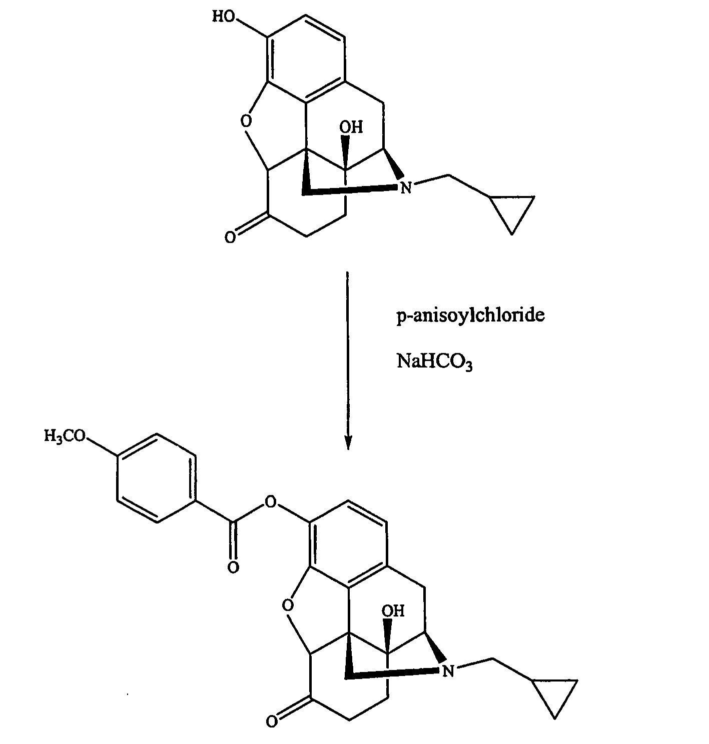

In a 2 liter 3-necked flask equipped with a magnetic stirrer was placed 20 g of Naltrexone HCl and 1.6 liters of 10% NaHCO₃. The mixture was placed in an oil bath set at 70°C and stirred mechanically. To the mixture was added 32 ml of p-anisoylchloride, dropwise at a rapid rate, via addition funnel. The reaction was monitored using thin-layer chromatography (TLC) (R_{f}= 0.5, 2.5%NH₃:MeOH/CH₂Cl₂) and was complete after 0.5 hours. The stirring was stopped, the reaction mixture allowed to settle, forming a semi-solid precipitate, and the aqueous phase was decanted from the precipitate. The precipitate was then dissolved in 500 ml of CH₂Cl₂. The resulting organic layer was washed with brine, and the organic layer was separated from the brine and dried (Na₂SO₄). The organic layer was concentrated under reduced pressure to provide a gelatinous residue. The gelatinous residue was dissolved in 30% hexane/CH₂Cl₂ and applied to a column packed with 200g of silica gel. The column was then eluted by stepwise elution with 1 liter of 30% hexane/CH₂Cl₂, 500 ml of CH₂Cl₂, and 1500 ml of 2%NH₃:MeOH/CH₂Cl₂. Fractions containing 3-(p-anisoylnaltrexone) were combined and the solvent fractions were concentrated to yield a viscous oil. 300ml of Et₂O were added to the viscous oil to provide a precipitate that was isolated by vacuum filtration and dried under high vacuum overnight to afford 10.8g of 3-(p-anisoylnaltrexone) as a white solid. The identity of the product was confirmed using electrospray mass spectrometry (ES/MS). ES/MS: expect 475.5, found 476.2[M+H]⁺, 508[M+33]⁺, 514.2[M+K]⁺. Analysis of the product using high-pressure liquid chromatography showed a single major peak (97% of total peak area).

3-(p-Anisoylnaltrexone) is converted to 3-(p-anisoylnaltrexone) hydrochloride by dissolving the 3-(p-anisoylnaltrexone) in an aprotic solvent, bubbling gaseous hydrochloric acid through the resulting solution to provide a precipitate of 3-(p-anisoylnaltrexone) hydrochloride, and collecting the 3-(p-anisoylnaltrexone) hydrochloride by filtration.

### Example 2: Synthesis of 3-palinitoylnaltrexone.

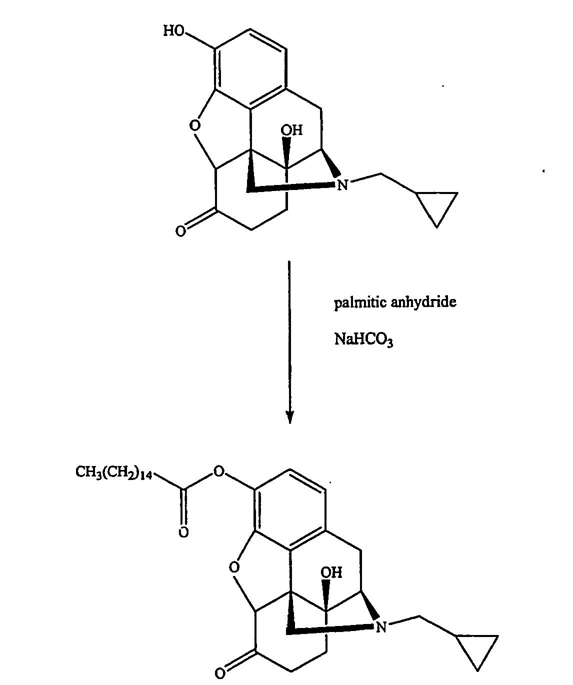

In a 2 liter 3-necked flask equipped with a mechanical stirrer was placed 10.8 g of naltrexone HCl, 770 ml of 10% NaHCO₃, 50 ml of CH₂Cl₂ and then 19.8 g of palmitic anhydride. The resulting reaction mixture was allowed to stir overnight at room temperature to provide a suspended solid. The suspended solid was isolated using vacuum filtration, and the isolated solid was dissolved in 500 ml CH₂Cl₂ and washed with brine. The CH₂Cl₂ layer was separated from the brine and dried (Na₂SO₄). The CH₂Cl₂ was evaporated to provide a semi-solid. The semi-solid was dissolved in CH₂Cl₂ and applied to a column packed with 225g of silica gel. The column was eluted using a step-wise elution with 1 liter of CH₂Cl₂ followed by 2 liters of 2% MeOH:NH₃/CH₂Cl₂. Fractions containing 3-palmitoylnaltrexone were combined, and the fractions were concentrated under reduced pressure to provide 15 g of a yellow oil that was essentially pure by TLC (R_{f}= 0.5, 2.5% MeOH:NH₃/CH₂Cl₂). To the yellow oil was added 100 ml of Et₂O. The resulting solution was placed into a freezer overnight to provide a white, soft, greasy solid that was isolated by decanting the mother liquor and evaporating excess solvent. The resulting white solid was then dried under high vacuum for 3 hours to provide 11.2 g of 3-palmitoylnaltrexone, whose identity was confirmed using electrospray mass spectrometry and elemental analysis for carbon, hydrogen, and nitrogen. ES/MS: expect 579.8, found 580.4[M+H]⁺, 612.6[M+33]⁺. Elemental analysis: theory C: 74.57, H: 9.21, N: 2.42; found C: 74.60, H: 9.31, N: 2.30. Analysis of the product by high pressure liquid chromatography showed a single major peak (99% of total peak area).

3-Palmitoylnaltrexone is converted to 3-palmitoylnaltrexone hydrochloride by dissolving the 3-palmitoylnaltrexone in an aprotic solvent, bubbling gaseous hydrochloric acid through the resulting solution to provide a precipitate of 3-palmitoylnaltrexone hydrochloride, and collecting the 3-palmitoylnaltrexone hydrochloride by filtration.

### Example 3: Synthesis of 3-(p-nitrobenzoyl)naltrexone, 3-(benzoyl)naltrexone, 3-(p-toluyl)naltrexone, and 3-(acetyl)naltrexone.

3-(p-Nitrobenzoyl)naltrexone, 3-(benzoyl)naltrexone, 3-(p-toluyl)naltrexone, and 3-(acetyl)naltrexone are made according to the procedure described above in Example 1 except that the p-anisoylchloride used in Example 1 is replaced with a corresponding equivalent of p-nitrobenzoyl chloride, benzoyl chloride, p-toluyl chloride, or acetyl chloride, respectively. The hydrochloride salts of 3-(p-nitrobenzoyl)naltrexone, 3-(benzoyl)naltrexone, 3-(p-toluyl)naltrexone, and 3-(acetyl)naltrexone are also made according to the procedure described above in Example 1.

### Example 4: Tamper-Resistant Transdermal-delivery Device. An aqueous gel is prepared from a mixture having the following formulation:

| Component | Percent by Weight |
|---|---|
| Ethanol | 22.1 |
| Hydroxyethylcellulose | 1.9 |
| Fentanyl (anhydrous) | 1.0 |
| The acyl opioid antagonist from Example 1, 2, or 3 (anhydrous) | 20.0 |
| Water | Balance |

A reservoir-type transdermal-delivery device having a drug releasing area of approximately 10 cm² is prepared by pouching, in a rotary heat sealing machine, the gel between an impermeable backing formed from an aluminized polyester/ethylene vinyl acetate copolymer (EVA) film (Scotchpak 1018, available from 3M Corporation) and a multi-laminate film comprising the rate controlling membrane, 2 mil ethylvinyl acetate (EVA) (9% vinyl acetate (VA)), 1.8 mil of an amine resistant silicone adhesive (Dow Corning X7920), and a fluorocarbon coated polyethylene terephthalate release liner (Scotchpak 1022, available from 3M Corporation) at a gel loading of approximately 15 mg/cm². The transdermal-delivery device is useful for treating or preventing pain in a patient.

### Example 5: Tamper-Resistant Transdermal-delivery Device.

An aqueous gel is prepared from a mixture having the following formulation:

| Component | Percent by Weigh | |
|---|---|---|
| | Range | Preperred |
| Ethanol | 20 - 90 | 75 |
| Hydroxyethylcellulose | 0.25 - 5 | 1.9 |
| Oxycodone (anhydrous) | 1 - 25 | 15 |
| The acyl opioid antagonist (anhydrous) | 1 - 25 | 5 |
| of Example 1, 2, or 3 | | |
| Water | Balance | Balance |

A transdermal-delivery device is prepared according to the procedure described above in Example 4 except that the gel used in Example 4 is replaced with the gel of this Example. The transdermal-delivery device is useful for treating or preventing pain in a patient. **Example 6:** Methods for Treating or Preventing Pain.

The transdermal-delivery device of Example 4 or 5 is contacted with a patient's skin for about 24 h and treats the patient's pain.

## Claims

1. A transdermal-delivery device comprising:
an analgesically effective amount of an opioid, or a pharmaceutically acceptable salt thereof; and
an acyl opioid antagonist, or a pharmaceutically acceptable salt thereof, in an amount sufficient to inhibit the euphoric effect of the opioid or pharmaceutically acceptable salt thereof.

2. The transdermal-delivery device of claim 1, wherein the amount of the opioid, or a pharmaceutically acceptable salt thereof, is from about 0.1 to about 500 mg and the molar ratio of the acyl opioid antagonist, or pharmaceutically acceptable salt thereof, to the opioid, or pharmaceutically acceptable salt thereof, is from about 1:16 to about 3:1.

3. The transdermal-delivery device of claim 1, wherein the transdermal-delivery device comprises a reservoir containing the opioid, or a pharmaceutically acceptable salt thereof, and the acyl opioid antagonist, or a pharmaceutically acceptable salt thereof.

4. The transdermal-delivery device of claim 3, further comprising a membrane adjacent to the reservoir.

5. The transdermal-delivery device of claim 1, wherein the transdermal-delivery device is a polymer-matrix type transdermal-delivery device.

6. The transdermal-delivery device of claim 1, wherein the transdermal-delivery device is a drug-in-adhesive type transdermal-delivery device.

7. The transdermal-delivery device of claim 1, wherein the opioid is selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dihydromorphone, dihydroisomorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl, heroin, hydrocodone, hydromorphone, hydromorphodone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, pantopon, papaveretum, paregoric, pentazocine, phenadoxone, phendimetrazine, phendimetrazone, phenomorphan, phenazocine, phenoperidine, piminodine, piritramide, propheptazine, promedol, properidine, propoxyphene, propylhexedrine, sufentanil, tilidine, tramadol, and a pharmaceutically acceptable salt thereof.

8. The transdermal-delivery device of claim 7, wherein the opioid is oxycodone or a pharmaceutically acceptable salt thereof.

9. The transdermal-delivery device of claim 7, wherein the opioid is hydrocodone or a pharmaceutically acceptable salt thereof.

10. The transdermal-delivery device of claim 7, wherein the opioid is fentanyl or a pharmaceutically acceptable salt thereof.

11. The transdermal-delivery device of claim 7, wherein the opioid is buprenorphine or a pharmaceutically acceptable salt thereof.

12. The transdermal-delivery device of claim 1, wherein the opioid antagonist is selected from the group consisting of cyclazocine, naloxone, naltrexone, nalmefene, nalbuphine, nalorphine, cyclazacine, levallorphan, and a pharmaceutically acceptable salt thereof.

13. The transdermal-delivery device of claim 12, wherein the opioid antagonist is naloxone, naltrexone, nalmefene, or a pharmaceutically acceptable salt thereof.

14. The transdermal-delivery device of claim 1, wherein the acyl is selected from the group consisting of (C₁-C₆)-C(O); phenylacetyl; tartaryl; glutamyl; succinyl; benzoyl that is unsubstituted or substituted with one or more C₁-C₃ alkyl, -CF₃, -NO₂, -halogen, or -O(C₁ - C₃ alkyl) groups; 4-hydroxybutyryl; glycolyl; lactyl; aspartyl; sulfanilyl; citryl; fumaryl; pamoyl; malyl; maleyl; hydroxymaleyl; p-toluenesulfonyl; steraryl; HOC(O)-R'-C(O), wherein R' is a C₃ - C₄ alkyl group; C₈-C₁₅ alkanoyl; C₈-C₁₈ alkenoyl; and 2-aminoacyl.

15. The transdermal-delivery device of claim 14, wherein the acyl is HOC(O)-R'-C(O), wherein R' is a C₃ - C₄ alkyl group.

16. The transdermal-delivery device of claim 1, wherein the acyl is an α-amino acid, a fatty acid or an aldonic acid, each having one or more carboxyl groups, less a hydroxyl group of one of the acid's carboxyl groups.

17. The transdermal-delivery device of claim 16, wherein the acyl is a fatty acid less a hydroxyl group of one of the fatty acid's carboxyl groups.

18. The use of a transdermal-delivery device as defined in any one of claims 1 through 17 in the manufacture of a product for treating or preventing pain in a patient, wherein said transdermal-delivery device is contacted with the skin of a patient for an amount of time sufficient to treat or prevent pain.

## Patentansprüche

1. Transdermale Verabreichungsvorrichtung, umfassend:
eine analgetisch wirksame Menge eines Opioids oder eine pharmazeutisch verträgliches Salz davon; und
einen Acyl-Opioid-Antagonisten oder ein pharmazeutisch verträgliches Salz davon in einer Menge, die ausreicht den euphorischen Effekt des Opioids oder pharmazeutisch verträglichen Salzes davon zu inhibieren.

2. Transdermale Verabreichungsvorrichtung gemäß Anspruch 1, wobei die Menge des Opioids oder des pharmazeutisch verträglichen Salzes davon von etwa 0,1 bis etwa 500 mg beträgt, und das molare Verhältnis des Acyl-Opioid-Antagonisten oder pharmazeutisch verträglichen Salzes davon zu dem Opioid oder pharmazeutisch verträglichen Salz davon von etwa 1:16 bis etwa 3:1 beträgt.

3. Transdermale Verabreichungsvorrichtung gemäß Anspruch 1, wobei die transdermale Verabreichungsvorrichtung ein Reservoir umfasst, welches das Opioid oder ein pharmazeutisch verträgliches Salz davon und den Acyl-Opioid-Antagonisten oder ein pharmazeutisch verträgliches Salz davon enthält.

4. Transdermale Verabreichungsvorrichtung gemäß Anspruch 3, weiterhin eine an das Reservoir anliegende Membran umfassend.

5. Transdermale Verabreichungsvorrichtung gemäß Anspruch 1, wobei die transdermale Verabreichungsvorrichtung eine transdermale Verabreichungsvorrichtung vom Polymer-Matrix-Typ ist.

6. Trasdermale Verabreichungsvorrichtung gemäß Anspruch 1, wobei die transdermale Verabreichungsvorrichtung eine Transdermale Verabreichungsvorrichtung vom Typ Arzneistoff im Klebstoff ist.

7. Transdermale Verabreichungsvorrichtung gemäß Anspruch 1, wobei das Opioid ausgewählt ist aus der Gruppe, bestehend aus Alfentanil, Allylprodin, Alphaprodin, Anileridin, Benzylmorphin, Bezitramid, Buprenorphin, Butorphanol, Clonitazen, Codein, Desomorphin, Dextromoramid, Dezocin, Diampromid, Diamorphon, Dihydrocodein, Dihydromorphin, Dihydromorphon, Dihydroisomorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Etophin, Dihydroetorphin, Fentanyl, Heroin, Hydrocodon, Hydromorphon, Hydromorphodon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Nalorphin, Nalbuphen, Normorphin, Norpipanon, Opium, Oxycodon, Oxymorphon, Pantopon, Papaveretum, Paregoric, Pentazocin, Phenadoxon, Phendimetrazin, Phendimetrazon, Phenomorphan, Phenazocin, Phenoperidin, Piminodin, Piritramid, Propheptazin, Promedol, Properidin, Propoxyphen, Propylhexedrin, Sufentanil, Tilidin, Tramadol und ein pharmazeutisch verträgliches Salz davon.

8. Transdermale Verabreichungsvorrichtung gemäß Anspruch 7, wobei das Opioid Oxycodon oder ein pharmazeutisch verträgliches Salz davon ist.

9. Transdermale Verabreichungsvorrichtung gemäß Anspruch 7, wobei das Opioid Hydrocodon oder ein pharmazeutisch verträgliches Salz davon ist.

10. Transdermale Verabreichungsvorrichtung gemäß Anspruch 7, wobei das Opioid Fentanyl oder ein pharmazeutisch verträgliches Salz davon ist.

11. Transdermale Verabreichungsvorrichtung gemäß Anspruch 7, wobei das Opioid Buprenorphin oder ein pharmazeutisch verträgliches Salz davon ist.

12. Transdermale Verabreichungsvorrichtung gemäß Anspruch 1, wobei der Opioid-Antagonist ausgewählt ist aus der Gruppe, bestehend aus Cyclazocin, Naloxon, Naltrexon, Nalmefen, Nalbuphin, Nalorphin, Cyclazacin, Levallorphan und ein pharmazeutisch verträgliches Salz davon.

13. Transdermale Verabreichungsvorrichtung gemäß Anspruch 12, wobei der Opioid-Antagonist Naloxon, Naltrexon, Nalmefen oder ein pharmazeutisch verträgliches Salz davon ist.

14. Transdermale Verabreichungsvorrichtung gemäß Anspruch 1, wobei das Acyl ausgewählt ist aus der Gruppe, bestehend aus (C₁-C₆)-C(O); Phenylacetyl; Tartaryl; Glutamyl; Succinyl; Benzoyl, welches unsubstituiert oder mit einer oder mehreren C₁-C₃-Alhyl-, CF₃-, NO₂-, Halogen- oder -O(C₁-C₃-Alkyl)-Gruppen substituiert ist; 4-Hydroxybutyryl; Glycolyl; Lactyl; Aspartyl; Sulfanilyl; Citryl; Fumaryl; Pamoyl; Malyl; Maleyl; Hydroxymaleyl; P-Toluenesulfonyl; Steraryl; HOC(O)-R'-C(O), worin R' eine C₃-C₄-Alkylgruppe ist; C₈-C₁₅ Alkanoyl; C₈-C₁₈ Akenoyl; und 2-Aminoacyl.

15. Transdermale Verabreichungsvorrichtung gemäß Anspruch 14, wobei das Acyl HOC(O)-R'-C(O) ist, wobei R' eine C₃-C₄-Alkylgruppe ist.

16. Transdermale Verabreichungsvorrichtung gemäß Anspruch 1, wobei das Acyl eine α-Aminosäure, eine Fettsäure oder eine Aldonsäure ist, welche jeweils eine oder mehrere Carboxylgruppen aufweisen, ohne eine Hydroxylgruppe von einer der Carboxylgruppen der Säure.

17. Transdermale Verabreichungsvorrichtung gemäß Anspruch 16, wobei das Acyl eine Fettsäure ohne eine Hydroxylgruppe von einer der Carboxylgruppen der Fettsäure ist.

18. Verwendung einer transdermalen Verabreichungsvorrichtung wie in einem der Ansprüche 1 bis 17 definiert zur Herstellung eines Produkts zur Behandlung oder Prävention von Schmerz bei einem Patienten, wobei die transdermale Verabreichungsvorrichtung die Haut eines Patienten für eine Zeitdauer kontaktiert, die ausreicht Schmerz zu behandeln oder zu verhüten.

## Revendications

1. Dispositif d'administration transdermique comprenant :
une quantité analgésiquement efficace d'un opioïde ou d'un sel pharmaceutiquement acceptable de celui-ci ; et
un antagoniste opioïde acyle ou un sel pharmaceutiquement acceptable de celui-ci, dans une quantité suffisante pour inhiber l'effet euphorique de l'opioïde ou d'un sel pharmaceutiquement acceptable de celui-ci.

2. Dispositif d'administration transdermique de la revendication 1, dans lequel la quantité de l'opioïde ou d'un sel pharmaceutiquement acceptable de celui-ci est d'environ 0,1 à environ 500 mg et le ratio molaire de l'antagoniste opioïde acyle ou d'un sel pharmaceutiquement acceptable de celui-ci, par rapport à l'opioïde ou à un sel pharmaceutiquement acceptable de celui-ci est d'environ 1:16 à environ 3:1.

3. Dispositif d'administration transdermique de la revendication 1, dans lequel le dispositif d'administration transdermique comprend un réservoir contenant l'opioïde ou un sel pharmaceutiquement acceptable de celui-ci ainsi que l'antagoniste opioïde acyle ou un sel pharmaceutiquement acceptable de celui-ci.

4. Dispositif d'administration transdermique de la revendication 3, comprenant en outre : une membrane adjacente au réservoir.

5. Dispositif d'administration transdermique de la revendication 1, dans lequel le dispositif d'administration transdermique est un dispositif d'administration transdermique à matrice polymère.

6. Dispositif d'administration transdermique de la revendication 1, dans lequel le dispositif d'administration transdermique est un dispositif d'administration transdermique de type médicament dans adhésif.

7. Dispositif d'administration transdermique de la revendication 1, dans lequel l'opioïde est sélectionné dans le groupe constitué de l'alfentanil, de l'allylprodine, de l'alphaprodine, de l'aniléridine, de la benzylmorphine, du bézitramide, de la buprénorphine, du butorphanol, du clonitazène, de la codéine, de la désomorphine, du dextromoramide, de la dézocine, du diampromide, de la diamorphone, de la dihydrocodéine, de la dihydromorphine, de la dihydromorphone, de la dihydroisomorphine, du diménoxadol, du dimépheptanol, du diméthylthiambutène, du butyrate de dioxaphétyle, de la dipipanone, de l'eptazocine, de l'éthoheptazine, de l'éthylméthylthiambutène, de l'éthylmorphine, de l'étonitazène, de l'étorphine, de la dihydroétorphine, du fentanyl, de l'héroïne, de l'hydrocodone, de l'hydromorphone, de l'hydromorphodone, de l'hydroxypéthidine, de l'isométhadone, de la cétobémidone, du lévorphanol, du lévophénacylmorphan, du lofentanil, de la mépéridine, du meptazinol, de la métazocine, de la méthadone, du métopon, de la morphine, de la myrophine, de la narcéine, de la nicomorphine, du norlévorphanol, de la norméthadone, de la nalorphine, du nalbuphène, de la normorphine, de la norpipanone, de l'opium, de l'oxycodone, de l'oxymorphone, du pantopon, du papaveretum, du parégorique, de la pentazocine, de la phénadoxone, de la phendimétrazine, de la phendimétrazone, du phénomorphan, de la phénazocine, de la phénapéridine, de la piminodine, du piritramide, de la propheptazine, du promédol, de la propéridine, du propoxyphène, de la propylhexédrine, du sufentanil, de la tilidine, du tramadol et d'un sel pharmaceutiquement acceptable de ceux-ci.

8. Dispositif d'administration transdermique de la revendication 7, dans lequel l'opioïde est l'oxycodone ou un sel pharmaceutiquement acceptable de celle-ci.

9. Dispositif d'administration transdermique de la revendication 7, dans lequel l'opioïde est l'hydrocodone ou un sel pharmaceutiquement acceptable de celle-ci.

10. Dispositif d'administration transdermique de la revendication 7, dans lequel l'opioïde est le fentanyl ou un sel pharmaceutiquement acceptable de celui-ci.

11. Dispositif d'administration transdermique de la revendication 7, dans lequel l'opioïde est la buprénorphine ou un sel pharmaceutiquement acceptable de celle-ci.

12. Dispositif d'administration transdermique de la revendication 1, dans lequel l'antagoniste opioïde est sélectionné dans le groupe constitué de la cyclazocine, de la naloxone, de la naltrexone, du nalméfène, de la nalbuphine, de la nalorphine, de la cyclazacine, du levallorphan et un sel pharmaceutiquement acceptable de ceux-ci.

13. Dispositif d'administration transdermique de la revendication 12, dans lequel l'antagoniste opioïde est la naloxone, la naltrexone, le nalméfène ou un sel pharmaceutiquement acceptable de ceux-ci.

14. Dispositif d'administration transdermique de la revendication 1, dans lequel l'acyle est sélectionné dans le groupe constitué de (C₁-C₆)-C(O) ; phénylacétyle ; tartaryle, glutamyle ; succinyle ; benzoyle, non substitué ou substitué avec un ou plusieurs groupes alkyles C₁-C₃, -CF₃, -NO₂, -halogène ou -O(alkyle C₁-C₃) ; 4-hydroxybutyryle ; glycolyle ; lactyle ; aspartyle ; sulfanilyle ; citryle ; fumaryle ; pamoyle ; malyle ; maléyle ; hydroxymaléyle ; p-toluènesulfonyle ; stéraryle ; HOC(O)-R'-C(O), dans lequel R' est un groupe alkyle C₃-C₄ ; alkanoyle C₈-C₁₅ ; alkénoyle C₈-C₁₈ ; et 2-aminoacyle.

15. Dispositif d'administration transdermique de la revendication 14, dans lequel l'acyle est HOC(O)-R'-C(O), dans lequel R' est un groupe alkyle C₃-C₄.

16. Dispositif d'administration transdermique de la revendication 1, dans lequel l'acyle est un acide α-aminé, un acide gras ou un acide aldonique, chacun comprenant un ou plusieurs groupes carboxyliques, amputé d'un groupe hydroxyle dans un des groupes carboxyliques de l'acide.

17. Dispositif d'administration transdermique de la revendication 16, dans lequel l'acyle est un acide gras amputé d'un groupe hydroxyle dans un des groupes carboxyliques de l'acide gras.

18. Utilisation d'un dispositif d'administration transdermique selon l'une des revendications 1 à 17 dans la fabrication d'un produit pour le traitement ou la prévention de la douleur chez un patient, dans laquelle ledit dispositif d'administration transdermique est mis en contact avec la peau d'un patient pendant un temps suffisant pour traiter ou prévenir la douleur.
